(19) **Europäisches Patentamt / European Patent Office / Office européen des brevets**

(11) **EP 1 758 470 B1**

(12) **EUROPEAN PATENT SPECIFICATION**

(45) Date of publication and mention
of the grant of the patent:
**30.07.2008 Bulletin 2008/31**

(51) Int Cl.:
*A23L 1/30* (2006.01)    *A23L 1/10* (2006.01)
*A61K 31/716* (2006.01)    *A61P 1/00* (2006.01)

(21) Application number: **05758958.2**

(22) Date of filing: **30.06.2005**

(86) International application number:
**PCT/BE2005/000105**

(87) International publication number:
**WO 2006/002495 (12.01.2006 Gazette 2006/02)**

(54) **PREBIOTIC PREPARATION**

PREBIOTISCHE ZUBEREITUNG

PREPARATION PREBIOTIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **30.06.2004 GB 0414655**

(43) Date of publication of application:
**07.03.2007 Bulletin 2007/10**

(73) Proprietor: **K.U. Leuven Research and Development**
**3000 Leuven (BE)**

(72) Inventors:
• **DELCOUR, Jan**
**B-3001 Heverlee (BE)**
• **COURTIN, Christophe**
**B-3012 Wilsele (BE)**
• **BROEKAERT, Willem**
**B-1700 Dilbeek (BE)**
• **SWENNEN, Katrien**
**B-3960 Opitter-Bree (BE)**

• **VERBEKE, Kristin**
**B-1910 Kampenhout (BE)**
• **RUTGEERS, Paul**
**B-3052 Blanden (BE)**

(74) Representative: **Bird, Ariane et al**
**Bird Goën & Co**
**Klein Dalenstraat 42A**
**3020 Winksele (BE)**

(56) References cited:
WO-A-02/067698    US-A- 5 362 502
US-A- 5 846 590    US-A1- 2002 037 331
US-A1- 2003 211 179    US-B1- 6 426 201

• VAN LAERE KMJ, HARTEMINK R, BOSVELD M, SCHOLS HA, VORAGEN AGJ: "Fermentation of plant cell wall derived polysaccharides and their corresponding oligosaccharides by intestinal bacteria" J. AGRIC. FOOD CHEM., vol. 48, 2000, pages 1644-1652, XP002344731

**Description**

**FIELD OF THE INVENTION**

The present invention relates to arabinoxylan preparations for use as prebiotic nutritional additives and to methods of improving gastro-intestinal health of human beings through the supplementation of their diets with the said additives. In a preferred embodiment, the arabinoxylan preparations are derived from natural sources, such as plant material and more preferably from cereals.

**BACKGROUND OF THE INVENTION**

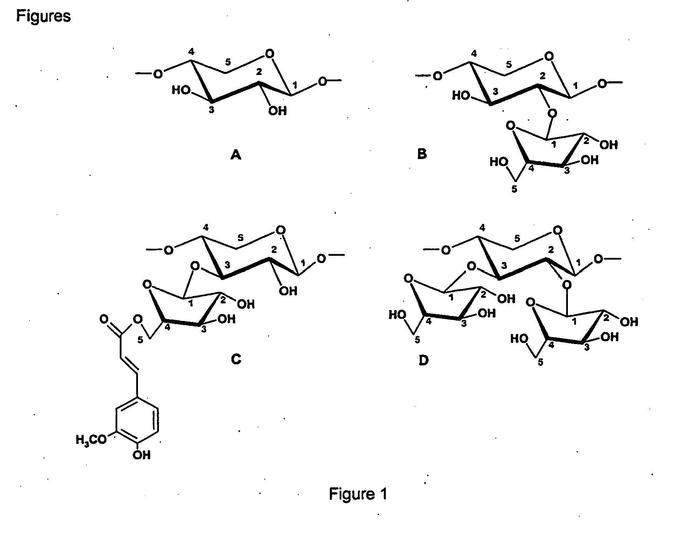

The invention relates to the positive effect on gastro-intestinal health, and more particularly on the gut microbiota, of food with given non-starch polysaccharides (NSP). NSP include a range of compounds possessing different physicochemical properties. Arabinoxylans, also called pentosans, are an important group of cereal NSP and consist of a main chain of β-1,4-linked D-xylopyranosyl units to which $O$-2 and/or $O$-3 α-L-arabino-furanosyl units are linked. In a typical arabinoxylan, unsubstituted, monosubstituted and disubstituted xylose residues occur (see Figure 1). Arabinoxylans are either water-extractable or water-unextractable. The latter can be partially solubilised under alkaline conditions or by using enzymes and bind large amounts of water. The water-extractable arabinoxylans have an extraordinary viscosity forming potential. In general, their molecular masses are very high (up to 800,000 Dalton) depending on the source and extraction method. Despite the fact that they are only minor constituents, they are important for the functionality of cereals in biotechnological processes such as the production of wheat starch, pasta and beer, breadmaking and other food applications.

Some types of oligosaccharides derived from arabinoxylan or xylan (an unsubstituted polymer of β-1,4-linked D-xylopyranosyl units) have been shown to exert prebiotic properties. Prebiotics are compounds, usually non-glucosidic oligosaccharides, that can not be digested by enzymes of the upper gastro-intestinal tract but are fermented selectively by some types of intestinal bacteria in the large intestine (Gibson and Roberfroid, 1995; Roberfroid, 1988; Van Loo, 2004). Presence of prebiotics in the diet causes a shift in the composition of the intestinal bacterial population, typically characterised by a relative increase in *Lactobacillus* and *Bifidobacterium* species. This shift in the microbiota of the intestine is associated with improved overall health, reduced gut infections, increased levels of intestinal short chain fatty acids, better absorption of minerals, and suppression of colon cancer initiation (Van Loo, 2004).

Preparations of xylo-oligosaccharides (XOS, oligosaccharides consisting of β-1,4-linked D-xylopyranosyl units) with predominance of oligosaccharides with a degree of polymerisation (DP) of 2-3 (xylobiose and xylotriose), have been shown to cause a significant increase in the level of Bifidobacteria in the faeces and caecum of rats (EP 0265970B1; Campbell et al., 1997; Hsu et al., 2004), and the colon of humans (Okazaki et al., 1990). Such xylobiose-rich XOS preparations also suppress early symptoms of chemical-induced colon carcinogenesis in rats (Hsu et al., 2004) and enhance the absorption of calcium (Toyoda et al., 1993). A preparation consisting predominantly of arabinoxylo-oligosaccharides (AXOS) with a DP of 3-5 (arabinosylxylobiose, arabinosylxylotriose, arabinosylxylotetraose, and diarabinosylxylotetraose) has also been shown to increase the levels of Bifidobacteria in the intestines of rats and mice (Yamada et al., 1993).

One major drawback of current commercial XOS preparations, which severely limits their commercial potential, is their very high price level compared to other oligosaccharides, indicating that current manufacturing processes are not cost-effective. A method has been described for the manufacturing of prebiotic preparations with predominance of XOS with DP 2-3, involving chemical extraction of xylan from plant-based products (hardwood, corn cobs, cottonseed hull, wheat bran, or brewery spent grain) using NaClO solutions and highly concentrated KOH solutions, followed by enzymic hydrolysis of the extracted xylan by endoxylanase enzymes (EP 0265970B1). A similar method has been used to make a prebiotic AXOS preparation (preparation of AXOS with DP 3-5) involving chemical extraction of arabinoxylan using a concentrated alkaline solution followed by removal of the salts, enzymic hydrolysis with endoxylanase, and chromatography on a carbon-column (Yamada et al., 1993). The main drawback of these methods is that the chemical extraction of xylan or arabinoxylan is environment-unfriendly, and requires costly removal of the chemicals by extensive dialysis or ultrafiltration before enzymic hydrolysis can be performed. Another method to produce XOS or AXOS involves hydrothermal autohydrolysis of hardwood or brewery spent grain. In this method a suspension of plant material is heated in a special reactor at 150-190°C for 20-60 min (EP 0265970B1; Kabel et al., 2002; Carvalheiro et al., 2004). The drawback of this method is that, due to the high reaction temperature, side products are produced that are undesirable for food purposes, such as furfural, hydroxymethylfurfural and levulinic acid (Carvalheiro et al., 2004).

The currently described prebiotic preparations of XOS and AXOS have an average degree of polymerisation that is either 2 (EP 026597081) or 4 (Yamada et al., 1993). For particular applications in the food sector these preparations have some drawbacks. First, the preparations are rich in xylose, which has a sweet taste that is about 60% as sweet as sucrose (Suntory, xylo-oligosaccharide, brochure and product sheet, 2001). Sweetness can be desired for some applications, but for other applications a more neutral taste is more desired. The xylo-oligosaccharides with a low average degree of polymerisation also have a sweet taste that is about 40% that of sucrose (Suntory, xylo-oligosaccharide, brochure and product sheet, 2001). Second, the preparations with a low average degree of polymerisation have an energy level that is not desired in low-calorie food ingredients. For the calculation of the energy value of xylo-oligosaccharides, the metabolisable energy value of xylose is considered 4 calories per g, 2 calories per g for xylobiose and xylotriose, and 0 cal per gram for xylo-arabino-oligosaccharides with a DP > 4 (Suntory, xylo-oligosaccharide, brochure and product sheet, 2001).

US 2002/037331 discloses arabinoxylans obtained from cereal bran which is extruded, enzymatically hydrolysed, and wherein the starch is hydrolysed. An ultrafiltration step with a cut-off of 5,000 is then carried out to purify the oligosaccharides thus obtained.

US-A-5,362,502 discloses the production of pentosans from farinaceous materials which may have a degree of polymerisation of about 50 or 17. WO 02/067698 discloses methods for the wet fractionation of cereal bran into two protein rich fractions, one of which contains the germ oils and related components, a fibre fraction, which also retains most of the aleurone proteins, and sugar syrup fraction.

## SUMMARY OF THE INVENTION

The present invention is defined by the claims. It relates to a nutritional additive to be used in the preparation of a food product or beverage which beneficially modulates the human intestinal flora, said additive comprising arabinoxylans having an average degree of polymerisation (DP) between 5 and 50. Furthermore, several food and beverage products comprising the additive are provided as well as methods to prepare the said additive.

## DETAILED DESCRIPTION

### *List of figures*

**Figure 1:** Structural elements of arabinoxylans. A: unsubstituted β-D-xylopyranosyl residue. **B**: β-D-xylopyranosyl residue substituted at *O*-2 with an α-L-arabinofuranosyl moiety. C: β-D-xylopyranosyl residue substituted at *O*-3 with an α-L-arabinofuranosyl moiety. D: β-D-xylopyranose residue substituted at *O*-2 and *O*-3 with α-L-arabinofuranosyl moieties. Structure C shows the linkage of ferulic acid to *O*-5 of an α-L-arabinofuranosyl residue.

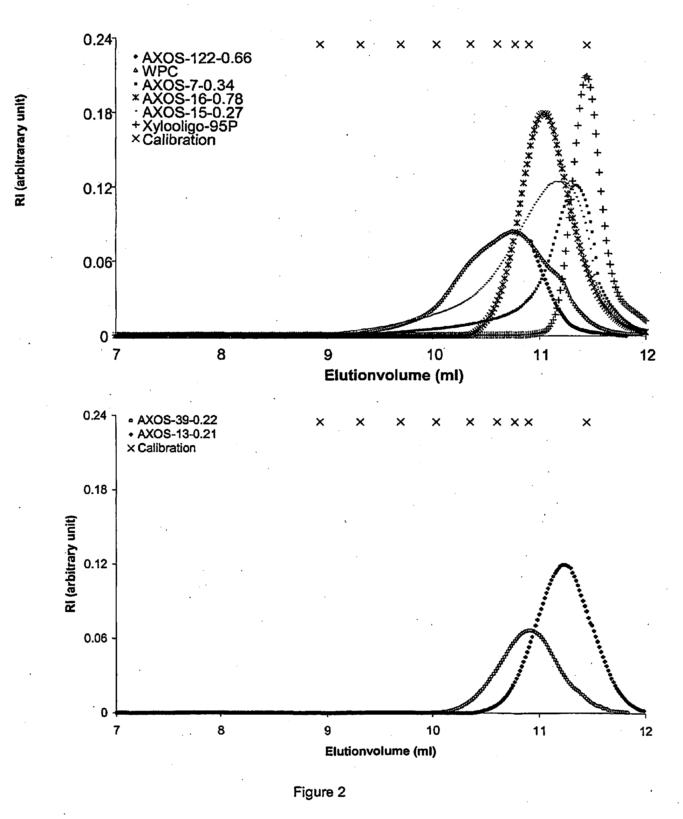

**Figure 2:** HPSEC molecular mass profiles of different AXOS preparations. The column was a Shodex SB-806 HQ (300 x 8 mm, Showa, Denko K.K., Tokyo, Japan). Elution volumes of pullulan standards with molecular mass of $78.8 \times 10^4$, $40.4 \times 10^4$, $21.2 \times 10^4$, $11.2 \times 10^4$, $4.73 \times 10^4$, $2.28 \times 10^4$, $1.18 \times 10^4$, $0.59 \times 10^4$ Da and of glucose (180 Da) are indicated by an "x" symbol from left to right.

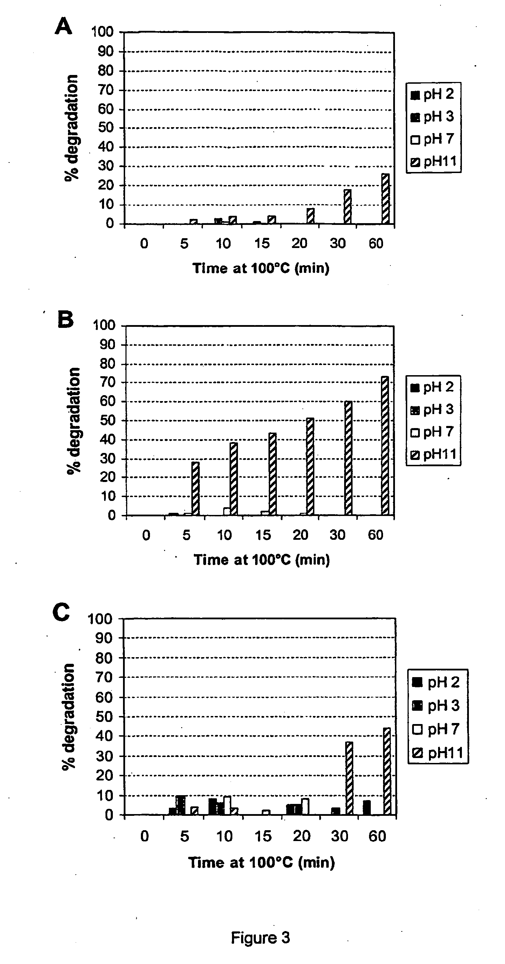

**Figure 3:** Percentage degradation of constituent monosaccharides of AXOS-15-0.27 (A). Xylooligo-95P (B), and Fructo-oligosaccharides (C) for different incubation times at 100°C at pH 2, 3, 7 and 11.

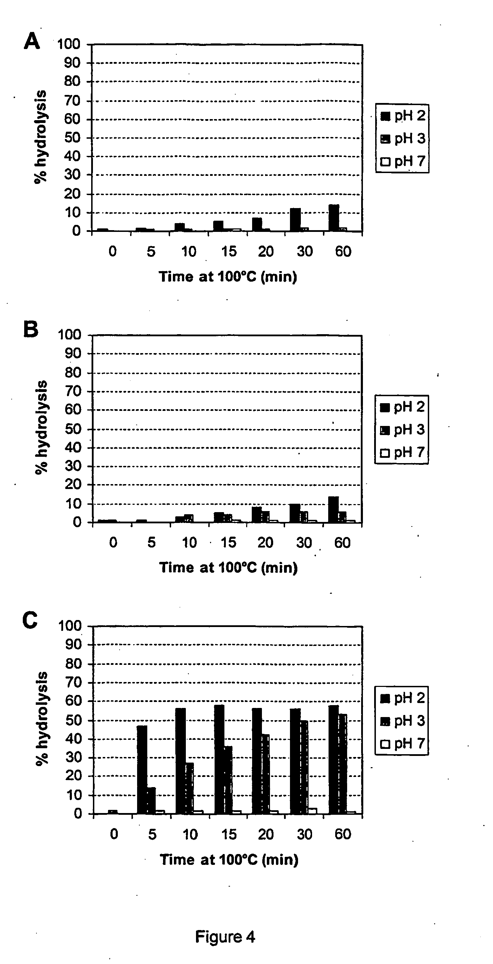

**Figure 4:** Percentage hydrolysis of AXOS-15-0.27 (A), Xylooligo-95P (B), and Fructo-oligosaccharides (C) for different incubation times at 100°C at pH 2, 3, and 7.

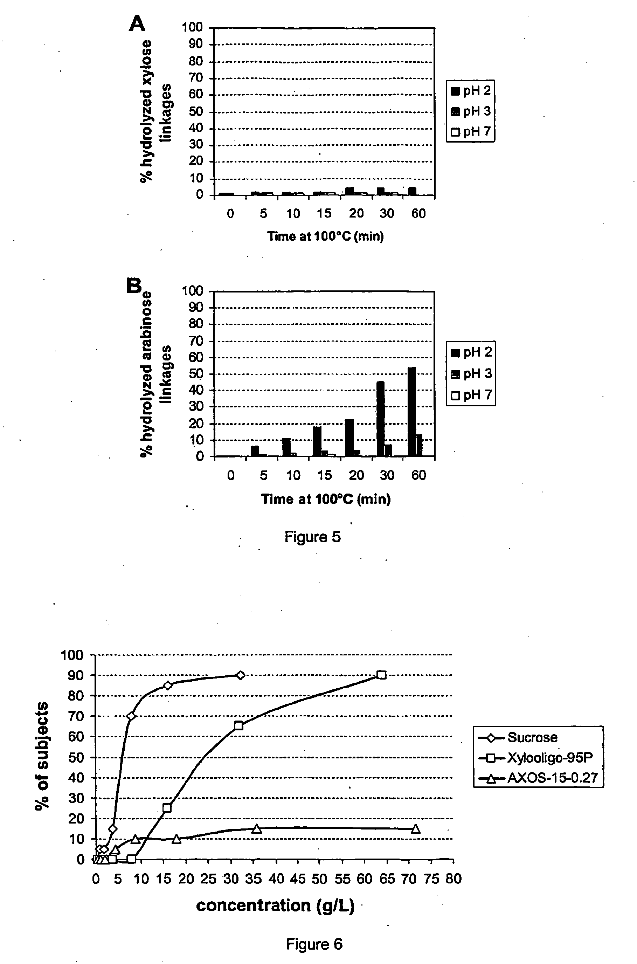

**Figure 5:** Percentage hydrolysis of xylose linkages (A) and arabinose linkages (B) in AXOS-15-0.27 for different incubation times at 100°C at pH 2, 3, and 7.

**Figure 6:** Sweetness of AXOS-15-0.27, Xylooligo-95P and sucrose. The curves show the cumulative percentage of the subjects (n = 20) recognizing sweet taste plotted against the concentration of the compound in g/l.

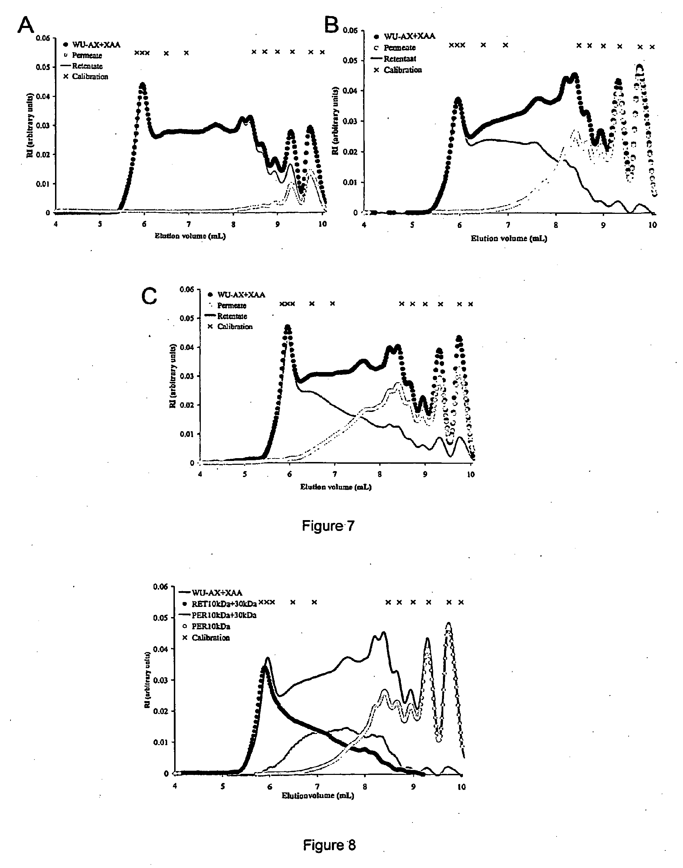

**Figure 7:** HPSEC molecular mass profiles of oligosaccharide fractions obtained after ultrafiltration of AXOS produced by endoxylanase treatment of squeegee WU-AX. The membranes used had a MMCO of 5 kDa (panel A), 10 kDa (panel B) and 30 kDa (panel C). The column was a Shodex SB-802.5 HQ (300 × 8 mm, Showa, Denko K.K., Tokyo, Japan). Molecular mass markers were Shodex standard P-82 pullulans with a molecular mass of $11.2 \times 10^4$, $4.73 \times 10^4$, $2.28 \times 10^4$, $1.18 \times 10^4$ and $0.59 \times 10^4$ Da, xylo-oligosaccharide standards with a molecular mass of 810 (DP6), 678 (DP5). 546 (DP4), 414 (DP3) and 282 Da (DP2) and glucose with a molecular mass of 180 Da, and their respective elution volume is indicated by an "x" symbol from left to right.

**Figure 8:** HPSEC molecular mass profiles of oligosaccharide fractions obtained after ultrafiltration by consecutive passing through membranes with MMCO of 10 kDa and 30 kDa of AXOS produced by endoxylanase treatment of squeegee WU-AX. Fractions analysed are either the retentate after passing through a 30 kDa MMCO of the retentate of a 10 kDa membrane ($RET_{10kDa+30kDa}$), the permeate after passing through a 30 kDa MMCO of the retentate of a 10 kDa membrane ($PER_{10kDa+30kDa}$), or the permeate after passing through a 10 kDa membrane ($PER_{10kDa}$). The column was a Shodex SB-802.5 HQ (300 x 8 mm, Showa, Denko K.K., Tokyo, Japan). Molecular mass markers were Shodex standard P-82 pullulans with a molecular mass of $11.2 \times 10^4$, $4.73 \times 10^4$, $2.28 \times 10^4$, $1.18 \times 10^4$ and $0.59 \times 10^4$ Da, xylo-oligosaccharide standards with a molecular mass of 810 (DP6), 678 (DP5), 546 (DP4), 414 (DP3) and 282 Da (DP2) and glucose with a molecular mass of 180 Da, and their respective elution volume is indicated by an "x" symbol from left to right.

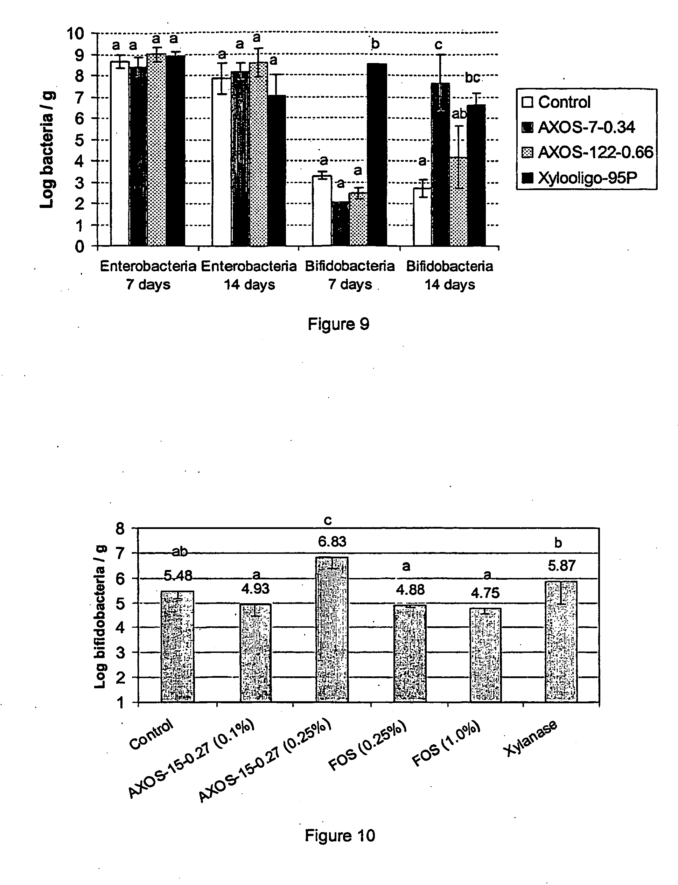

**Figure 9**: Effects of the addition to chicken feed of AXOS-7-0.34, AXOS-122-0.66 or Xylooligo-95P on the microbiota In the caecum of chickens. The composition of the caecal microbiota was determined 1 and 2 weeks, respectively, after the start of the experiment by plate counting for anterobacteriaciae and bifidobacteriaceae. Bars represent averages of the measurements and error bars indicate the standard deviation. For a given time point the values marked with a different letter are significantly different from each other according to Tukey's test at $p < 0.05$.

**Figure 10:** Effects of the addition to chicken feed of AXOS-15-0.27 at 0.1% or at 0.25%, Fructo-oligosaccharides (FOS) at 0.25% or 1%, or endoxylanase on the number of bifidobacteria in the caecum of chickens after 14 days. Bacteria belonging to the genus Bifidobacterium were measured by quantitative PCR. The values marked with a different letter are significantly different from each other according to the least significance difference test ($p < 0.05$; $n = 3$). Bars represent averages of the measurements and error bars indicate the standard deviation.

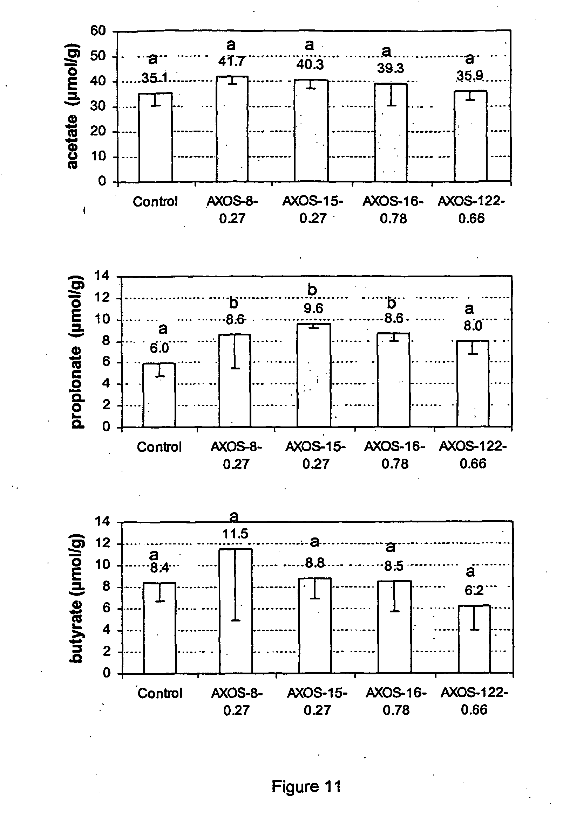

**Figure 11:** Effects of the addition to rat diets of 0.25% of AXOS-8-0.27, AXOS-15-0.27, AXOS-16-0.78 or AXOS-122-0.66 on the level of acetate (top panel), propionate (middle panel) and butyrate (bottom panel) in faeces of rats after 13 days of feeding. The values marked with a different letter are significantly different from each other according to the least significance difference test ($p < 0.05$; $n = 4$). Bars represent averages of the measurements and error bars indicate the standard deviation.

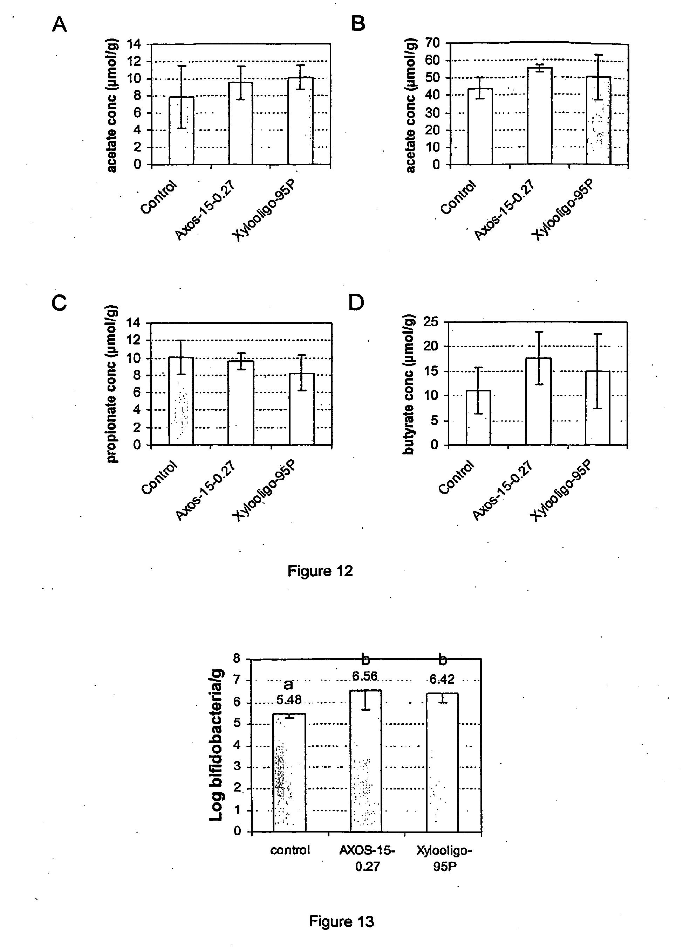

**Figure 12:** Effects of the addition to rat diets of 4% AXOS-15-0.27 or Xylooligo-95P on the level of acetate in the proximal colon (A), acetate in the distal colon (B), propionate in the distal colon (C) and butyrate in the distal colon (D) of rats after 14 days of feeding. Bars represent averages of the measurements and error bars indicate the standard deviation.

**Figure 13:** Effects of the addition to rat diets of 4% AXOS-15-0.27 or Xylooligo-95P on the level of bifidobacteria in the caecum after 14 days of feeding. Bacteria belonging to the genus Bifidobacterium were measured by quantitative PCR. The values indicated with a different letter are significantly different from each other according to the least significance difference test ($p < 0.05$; $n = 4$). Bars represent averages of the measurements and error bars indicate the standard deviation.

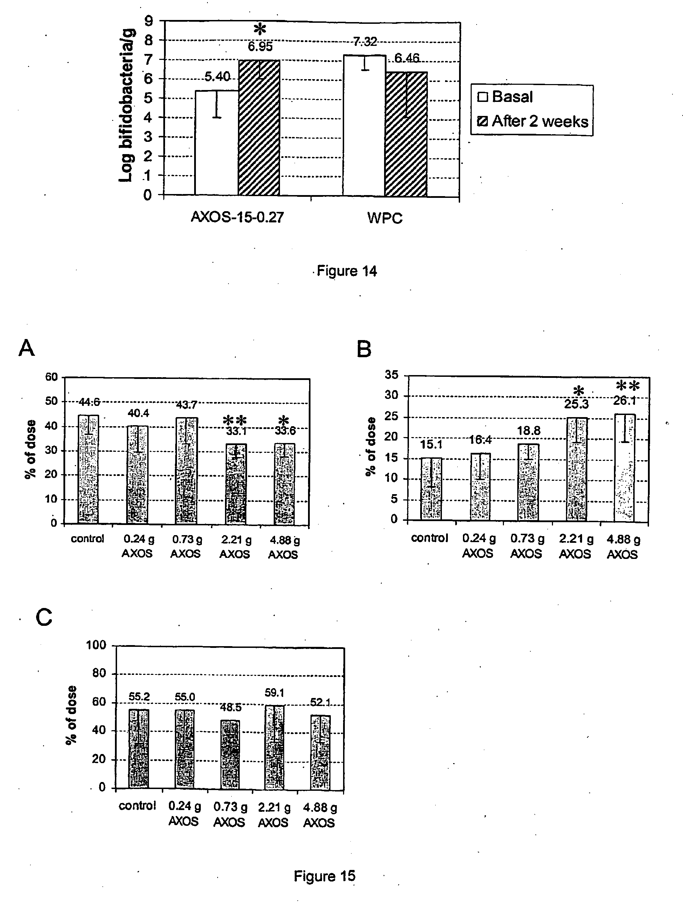

**Figure 14:** Effect of the ingestion of 4.88 g/day of AXOS-15-0.27 or 4.81 g/day of WPC during 14 days on the number of bifidobacteria in the faeces of healthy human volunteers. Bacteria belonging to the genus Bifidobacterium were measured by quantitative PCR. Bars represent averages of the measurements and error bars indicate the standard deviation. Bars with a star show a significant difference from the basal level according to the Wilcoxon Signed Ranks test ($p < 0.05$; $n = 5$).

**Figure 15:** Effect of the ingestion of once-off doses of AXOS-15-0.27 (0.24, 0.73, 2.21 or 4.88 g) on urinary (A) and faecal (B) excretion of nitrogen, and on oro-caecal transit time (C). Nitrogen excretion is expressed as the percentage of the administered $^{15}$N-labeled nitrogen recovered in either the urine or faeces samples collected during 0-48h and 0-72h after ingestion of the test meal, respectively. Oro-caecal transit time is expressed as the percentage of the administered $^3$H-labelled PEG in faecal samples collected during 0-72h. Bars represent averages of the measurements and error bars indicate the standard deviation. Bars with one star or two stars show a significant difference from the control treatment according to the to the Wilcoxon Signed Ranks test at $p < 0.05$ ($n = 9$) and $p < 0.01$ ($n = 9$), respectively.

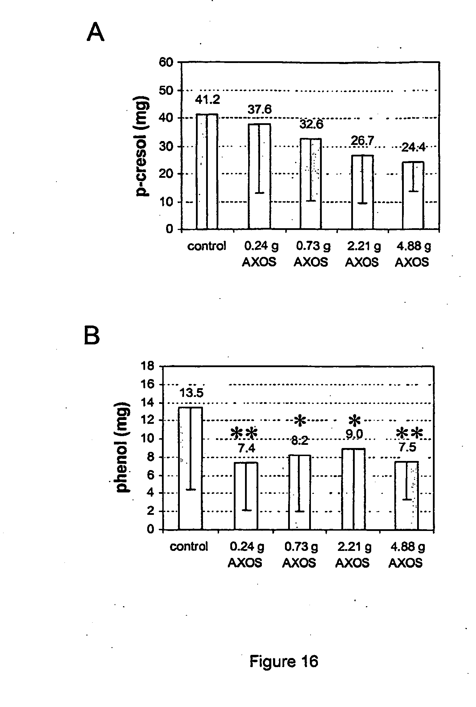

**Figure 16:** Effect of the ingestion of once-off doses of AXOS-15-0.27 (0.24, 0.73, 2.21 or 4.88 g) on urinary p-cresol (A) and phenol (B) excretion. p-Cresol and phenol excretion are expressed as the total p-cresol and phenol content (mg) recovered in urine samples collected over 0-24h after ingestion of the test meal. Bars represent averages of the measurements and error bars indicate the standard deviation. Bars with one star or two stars show a significant difference from the control treatment according to the to the Wilcoxon Signed Ranks test at $p < 0.05$ (n = 9) and $p<0.01$ (n = 9), respectively.

*Description*

The prebiotic effect of supplementing feed or food products with particular arabinoxylan preparations has been described previously. More particularly the prior art provides examples of the bifidogenic effects of on the one hand xylo-arabino-oligosaccharides having an average degree of polymerisation (DP) lower than 4 and on the other hand of arabinoxylan preparations comprising long chain naturally occurring arabinoxylans.

The available xylo-arabino-oligosaccharide preparations are produced under drastic and uncontrolled depolymerisation conditions resulting in the presence of relatively high levels of xylose and short xylo-oligosaccharides, which have a sweet taste. The sweetness of these preparations limits their use to specific food products. Furthermore, xylo-arabino-oligosaccharide molecules with a DP lower than 4 have a metabolical energy content, which is undesired in low-calorie foods.

The physicochemical characteristics of naturally occurring arabinoxylans, such as high viscosity and high water retention, make them unsuited for use in a wide range of food products. Partial depolymerisation of natural arabinoxylans improves their physicochemical characteristics and preparations comprising arabinoxylan molecules having an average DP of about 58 have been described. However, the prebiotic action in humans of these partially depolymerised arabinoxylans has only been demonstrated at relatively high doses (> 6 g per day; Grasten et al. 2003).

The present invention is based on the finding that arabinoxylan preparations comprising arabinoxylans having an average DP between 5 and 50 are potent prebiotic agents when administered to human beings and test animals serving as models for humans. Moreover, such preparations have none of the physicochemical or organoleptic inconveniences of the preparations comprising either natural arabinoxylans or short-chain xylo-arabino-oligosaccharides. It was also found that in both humans, rats and chickens the arabinoxylan preparations of the present invention had a stronger bifidogenic effect than preparations comprising partially depolymerised arabinoxylans having an average DP of 58 or more. In addition it was observed that the arabinoxylan preparations of the present invention mainly exert their prebiotic action in the distal part of the colon, while short-chain xylo-oligosaccharides were more active in the proximal part of the colon. This finding is particularly important as their appears to be a relation between the composition of the flora in the distal colon and the development of colon disease and more particularly colon cancer.

Next to the prebiotic effect *per se* also other beneficial effects of the use of the arabinoxylan preparations of the present invention were seen such as a reduction of urinary nitrogen excretion and concomitant increase of faecal N-excretion, as well as a reduction of the urinary excretion of cresol and phenol.

Therefore, in a first object the present invention provides the use of an arabinoxylan preparation comprising arabinoxylan molecules with an average degree of polymerisation (DP) varying between 5 and 50 as a food additive in the production of a food or beverage which comprises between 0.25 and 5 g of such arabinoxylans per serving of said food or beverage ,or as a basis for a nutritional supplement product. In a preferred embodiment the arabinoxylan preparation comprises at least 15% of said arabinoxylan molecules. In a more preferred embodiment the preparations comprises more than 30% of said arabinoxylans, for instance more than 60%. In an even more preferred embodiment the arabinoxylans comprised in preparations according to the present invention have an average DP between 7 and 40 DP, even more preferably between 7 and 20 DP. In a particular embodiment the present invention provides the use of an arabinoxylan preparation comprising arabinoxylan molecules with an average degree of polymerisation (DP) of 5 or 7, or an average degree of polymerisation (DP) of 6 when their A/X (arabinose to xylose) ratio is 0.40, or an average degree of polymerisation (DP) of 8 when their A/X (arabinose to xylose) ratio is 0.50, as a food additive in the production of a food or beverage which comprises between 0.25 and 5 g of such arabinoxylans per serving of said food or beverage. Preferably, 90% of the arabinoxylans comprised in the preparations of the present invention have a DP between 2 and 650 as determined using High Performance Size Exclusion Chromatography (HPSEC), more preferably between 2 and 130.

In a preferred embodiment, the arabinoxylan preparations of the present invention are obtainable from natural sources, such as plant material and more preferably from cereals. They can be selected fractions of said natural arabinoxylans or can be obtained by depolymerisation or fragmentation of said natural arabinoxylans or they can be structural analogues produced by chemical, enzymic and/or physical processes. In a preferred embodiment the arabinoxylan preparations are derived from a side-stream of the gluten-starch separation process, such as WPC (Pfeifer & Langen). In another preferred embodiment the arabinoxylan preparations are derived from cereal bran.

Prebiotic effects of the arabinoxylan preparations of the present invention when administered via the food were observed at a dose of 0.25 g per serving. So in a second object the present invention provides a food or beverage product modulating the human intestinal flora, comprising between 0.25 and 5 g of arabinoxylans having an average DP between 5 and 50 per serving of said food or beverage product. In a particular embodiment the present invention provides a food or beverage product modulating the human intestinal flora comprising between 0.25 and 5 g of arabinoxylans per serving of said food or beverage product, wherein said arabinoxylans have an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50. In an even more preferred embodiment the food or beverage product comprises between 1 and 3 g of arabinoxylans having an average DP between 5 and 50 per serving. As indicated above arabinoxylan preparations according to the present invention are particularly suited as a beneficial additive to low calorie foods.

In a particular embodiment the food product is a dairy product such as yoghurt or fresh cheese. Preferably said dairy product comprises between 0.25 and 5 g, more preferably between 1 and 3 g, of arabinoxylans having an average DP between 5 and 50 per 100 g per serving of 125 g. Optionally said dairy product comprises living bacteria of the genus Bifidobacterium or Lactobacillus, which are capable of fermenting arabinoxylans. In another particular embodiment the beverages of the present invention are so called non-alcoholic functional soft drinks. Preferably such functional soft drinks comprise between 0.25 and 5 g, more preferably between 1 and 3 g, of arabinoxylans having an average DP between 5 and 50 per 100 ml. Alternatively said functional soft drink comprises the desired amount of such arabinoxylans in 250 ml. In a particular embodiment said functional soft drinks comprise living bacteria of the genus Bifidobacterium or Lactobacillus, which are capable of fermenting arabinoxylans.

In general, food products having cereals or cereal derived material as an ingredient contain arabinoxylans. However, the arabinoxylans comprised in these food products are either long-chain natural arabinoxylans with a DP of over 6000 or partially depolymerised arabinoxylans having a DP of at least 200 to 300. Therefore, the enrichment of said food products with arabinoxylans according to present invention also improves their nutritional value. Preferably, such enrichment is obtained by adding a given amount of an arabinoxylan preparation of the present invention as an ingredient during the preparation of cereal-containing food products. It is clear that such enrichment results in a cereal-containing food product comprising arabinoxylans having a DP of 200 and higher, next to a population of between 0.25 and 5 g of arabinoxylans having a DP below 200 per serving of said food product, said population of arabinoxylans having an average DP between 5 and 50. In a particular embodiment the present invention provides a cereal-containing food product comprising between 0.25 and 5 g of arabinoxylans having a DP below 200 per serving of said product and wherein said population of arabinoxylans has an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50. The person skilled in the art will understand that the enrichment of the cereal-containing food products with the said population of arabinoxylans having a DP below 200 can at least in part be obtained by using xylanolitic enzymes under appropriate conditions during the preparation of these food products. In a particular embodiment said food product is a bakery product such as bread. Preferably said bread is enriched with between 0.25 and 5 g, more preferably between 1 and 3 g, of arabinoxylans having an average DP between 5 and 50 per 100 g. In another embodiment the food product is a pastry product, such as cake. Preferably said pastry product is enriched with between 0.25 and 5 g, more preferably between 1 and 3 g, of arabinoxylans having an average DP between 5 and 50 per 100 g. In yet another embodiment the food product is a pasta product Preferably said pasta product is enriched with between 0.25 and 5 g, more preferably between 1 and 3 g, of arabinoxylans having an average DP between 5 and 50 per 80 g. In yet another embodiment the food product is a breakfast cereal. Preferably said breakfast cereal is enriched with between 0.25 and 5 g, more preferably between 1 and 3 g, of arabinoxylans having an average DP between 5 and 50 per 30 g. In yet another embodiment the food product is a biscuit, for instance dry breakfast biscuit. Preferably said biscuit is enriched with between 0.25 and 5 g, more preferably between and 3 g, of arabinoxylans having an average DP between 5 and 50 per 125 g.

Other food products that can beneficially be supplemented with the arabinoxylan preparations of the present invention are for example, but without limitation, ground meat products, chocolates, cookies, bars, and desserts such as dessert puddings.

In a third object the present invention provides a nutritional additive to be used as an additive in the preparation of a food product or beverage according to the present invention, wherein said additive comprises at least 15% arabinoxylans having an average degree of polymerisation (DP) between 7 and 20. In a particular embodiment the present invention provides a nutritional additive to be used as an additive in the preparation of a food product or beverage according to the invention, wherein said additive comprises at least 15% arabinoxylans, wherein said arabinoxylans have an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50. In a preferred embodiment the food supplement product is a capsule, tablet, powder or the like. In a more preferred embodiment the food supplement product is formulated such that it allows a daily administration of between 0.25 and 5 g of arabinoxylans having an average DP between 5 and 50, , for instance between 1 and 3 g.

In a fourth object the present invention provides methods to prepare a nutritional additive to be used as an additive in the preparation of a food product according to the present invention. In a first embodiment the method comprises the steps of:

i. isolating the water unextractable arabinoxylan fraction from an arabinoxylan-rich material such as bran, wheat flour or wheat squeegee by destarchification and deproteinisation of said arabinoxylan rich material, preferably using amylolytic and proteolytic enzymes, respectively
ii. depolymerising the water unextractable arabinoxylan fraction using one or more xylanolitic enzymes

In a second embodiment the method comprises the steps of:

i. obtaining a partially depolymerised water extractable arabinoxylan-containing preparation, such as a preparation obtained out of a side stream of the starch-gluten separation process
ii. if necessary eliminating the starch and proteins from said arabinoxylan-containing preparation, preferably using amylolytic and proteolytic enzymes, respectively,
iii. depolymerising the arabinoxylans contained in said preparation using one or more xylanolitic enzymes

In a particular embodiment the preparation is subjected to ultrafiltration after the treatment with the xylanolitic enzymes in order to reduce the amount of monosaccharides and oligosaccharides having a DP of 4 or less from the preparation.

In a final object the present invention provides a method for the analysis of the concentration and average DP of a population of arabinoxylans with a DP smaller than 200 in a cereal containing-food product. Said method comprising the steps of:

i. grinding a sample of the cereal-containing food product, preferably after having dried or freeze-dried said sample;
ii. extracting the ground sample in distilled water;
iii. treating the extract with amylolytic enzymes at appropriate conditions in order to transform all starch into glucose, for instance using an amylase treatment followed by an amyloglucosidase treatment;
iv. eliminating the glucose from the sample obtained in (iii), for instance by metabolising the glucose using yeast;
v. determining the molecular mass profile of the arabinoxylans comprised in the sample obtained in (iv), preferably using High Performance Liquid Chromatography (HPLC);
vi. calculating the relative amount and average DP of the arabinoxylan fraction smaller than DP 200, for instance by analysis of the surface under the HPLC curve.

The invention is further illustrated by way of the illustrative embodiments described below.

## EXAMPLES

### EXAMPLE 1: Preparation of XOS/AXOS with average DP >4

Materials and methods

*Xylooligo-95P.* The commercial xylo-oligosaccharide preparation Xylooligo-95P, consisting predominantly of xylobiose, xylotriose, and xylótetraose, was obtained from Suntory Ltd. (Tokyo, Japan).

*Preparation of water-unextractable arabinoxylan from wheat bran.* For the preparation of bran water-unextractable arabinoxylan (WU-AX), commercial wheat bran (Meneba Meel BV, Rotterdam, The Netherlands) was used as starting material. Non-arabinoxylan material was partially removed from the bran by enzymic treatment according to the method described in Maes et al. (2004). A suspension of wheat bran in water (1:7 w/v) was first treated with a thermostable $\alpha$-amylase (Termamyl 120LS, Novozymes, Bagsvaerd, Denmark; 1 $\mu$l/g wheat bran) for 90 min at 90°C to hydrolyse the starch. After cooling to 50°C, the pH of the suspension was adjusted to 6.0 using concentrated HCl and the suspension was incubated with a protease (Neutrase 0.8L, Novozymes, Bagsvaerd, Denmark; 150 $\mu$l/g wheat bran) for 24 h at 50°C to hydrolyse residual proteins. Thereafter, the suspension was boiled and filtered and the filtrate discarded. The residue, referred to as "bran WU-AX", was washed with water and air-dried.

*Preparation of water-unextractable arabinoxylan from wheat flour or wheat squeegee starch.* Wheat flour can be fractionated in 4 different fractions using standard methods (McRitchie, 1985): A-starch (prime starch), B-starch (squeegee starch or tailings), gluten and a water soluble fraction. The WU-AX is concentrated in the squeegee starch fraction. Non-arabinoxylan material was partially removed from the squeegee fraction by enzymic treatment. A suspension of squeegee starch in water (1:6 w/v) was first treated with a thermostable $\alpha$-amylase (Termamyl 120LS, Novozymes, Bagsvaerd, Denmark, 30 $\mu$l/g squeegee starch) for 60 min at 90°C and amyloglucosidase (Megazyme, Bray, Ireland, 20 $\mu$l/g squeegee starch) for 16 h at 60°C to hydrolyse the starch. After boiling for 30 minutes and centrifugation, the residue was incubated with a protease (Neutrase 0.8L, Novozymes, Bagsvaerd, Denmark, 20 μl/g squeegee starch) for 20 h at 50°C to hydrolyse residual proteins. Thereafter, the suspension was boiled again (30 min), filtered and the filtrate discarded. The residue, referred to as "flour WU-AX", was washed with water, ethanol (95% v/v) and air-dried. Rather than extracting WU-AX from wheat starch, WU-AX can also be directly isolated from wheat squeegee starch. In this case, Meritena 233 (Tate & Lyle, Aalst, Belgium), a commercially available side-stream of the industrial starch-gluten separation process, was used as starting material. A suspension of Meritena 233 in water (1:5 w/v) was subsequently treated with a thermostable α-amylase (Termamyl 120LS, Novozymes, Bagsvaerd, Denmark; 30 μl/g Meritena 233) for 60 min at 90°C and amyloglucosidase (Megazyme, Bray, Ireland; 20 μl/g Meritena 233) for 16 h at 60°C to hydrolyse the starch. After boiling for 30 minutes and centrifugation, the residue was incubated with a protease (Neutrase 0.8L, Novozymes, Bagsvaerd, Denmark; 20 μl/g Meritena 233) for 20 h at 50°C to hydrolyse residual proteins. Thereafter, the suspension was boiled again (30 min), filtered and the filtrate discarded. The residue, referred to as "squeegee WU-AX", was washed with water, ethanol (95% v/v) and air-dried.

*Wheat Pentosan Concentrate (WPC).* Wheat Pentosan Concentrate (WPC, Pfeifer & Langen, Dormagen, Germany) is derived from wheat flour and its chemical composition has been described in detail by Courtin and Delcour (1998). WPC is rich in water extractable arabinoxylan (ca. 43%) and protein material (ca. 30%). The remaining part mainly consists of arabinogalactan peptide (ca. 14%) and to a lesser extent, polymeric glucose (6%).

*Preparation of AXOS with avDP of 122 and A/X ratio of 0. 66 (AXOS-122-0.66).* The starting material for the preparation of AXOS-122-0.66 was the commercial product Wheat Pentosan Concentrate (WPC, Pfeifer & Langen, Dormagen, Germany). The WPC was solubilised in deionised water (1:10 w/v) and silica was added as an aqueous suspension (20% w/v) until a silica/protein ration of 7:1. De pH of the mixture was adjusted to 4.8 using 0.1 M HCl in order to obtain a maximal adsorption of the proteins to the silica. After 30 min stirring the suspension was Büchner filtered. The residue comprising the silica/protein was discarded, while the filtrate was subjected to an ethanol precipitation. Ethanol (95% v/v) was added under continuous stirring to a final concentration of 65% (v/v) and after stirring for an additional 30 min, settling (24 h, 4°C) and filtration, the obtained residue, was solvent-dried (ethanol, acetone and diethyl ether) and air-dried. The obtained material was homogenised and sieved through 250 μm sieve.

*Preparation of AXOS with avDP of 16 and A/X ratio* of *0.78 (AXOS-16-0.78).* AXOS-16-0.78 was prepared starting from the commercial product Wheat Pentosan Concentrate (WPC, Pfeifer & Langen, Dormagen, Germany). WPC was treated with silica to remove proteins as described for the preparation of AXOS-122-0.66. The recovered filtrate was further incubated at 30°C during 24 h with an XAA, an endoxylanase from *Aspergillus aculeatus* (Shearzyme 500L, Novozymes, Bagsvaerd, Denmark) at 29 units per g Wheat Pentosan Concentrate. After inactivation of the enzyme by boiling (30 min), the obtained solution was cooled and subjected to an ethanol precipitation. Ethanol (95% v/v) was added under continuous stirring to a final concentration of 80% (v/v) and after stirring for an additional 30 min, settling (24 h, 4°C) and filtration, the obtained residue was dissolved in deionised water and again subjected to an ethanol precipitation. Ethanol (95% v/v) was added under continuous stirring to a final concentration of 65% (v/v) and after stirring for an additional 30 min, settling (24 h, 4°C) and filtration, the precipitated material was removed. The remaining supernatant was subjected to rotary evaporation, to remove ethanol, dissolved in deionised water and lyophilised. The obtained material was homogenised and sieved through a 250 μm sieve.

*Preparation of AXOS with avDP of 7 and A/X ratio of 0.34 (AXOS-7-0.34).* The starting material for the preparation of AXOS-7-0.34 was bran WU-AX that was isolated as described above. Bran WU-AX was subsequently incubated at 30°C during 24 h with an endoxylanase XBS (Grindamyl H640, Danisco, Denmark) at 80 units per g dry isolated bran WU-AX. After filtration and inactivation of the enzyme by boiling (30 min), the filtrate was lyophilised and the obtained material was homogenised and sieved through a 250 μm sieve.

*Preparation of AXOS with avDP* of 15 *and A/X ratio of 0.27 (AXOS-15-0.27).* Commercial wheat bran (Dossche Mills & Bakery, Deinze, Belgium) was used as starting material for the preparation of AXOS-15-0.27. A suspension of wheat bran in water (1:7 w/v) was first treated with a thermostable α-amylase (Termamyl 120LS, Novozymes, Bagsvaerd, Denmark; 1 μl/g wheat bran) for 90 min at 90°C to hydrolyse the starch. After cooling to 50°C, the pH of the suspension was adjusted to 6.0 using concentrated HCl and the suspension was incubated with a protease (Neutrase 0.8L, Novozymes, Bagsvaerd, Denmark; 40 μl/g wheat bran) for 4 h at 50°C to hydrolyse residual proteins. Thereafter, the suspension was boiled during 20 min, filtered and the filtrate discarded. The residue was washed with water, and resuspended in deionised water (1:14 w/v). The suspension was incubated under continuous stirring for 10 h at 50°C with endoxylanase XBS (Grindamyl H640, Danisco, Denmark) at 1.4 units per g destarched and deproteinised wheat bran, and for another 10 h at 50°C after addition of a second dose of endoxylanase XBS at 1.1 units per g destarched and deproteinised wheat bran. After inactivation of the enzyme by boiling (30 min), the solution was concentrated till 20% dry matter in a falling film evaporator and finally dried in a spray-drier.

*Preparation of AXOS with avDP of 8 and A/X ratio of 0.27 (AXOS-B-0.27).* AXOS-8-0.27 was prepared by incubating a solution (1:10 w/v) of AXOS-15-0.27 at 30°C during 1 h with XAA, an endoxylanase from *Aspergillus aculeatus* (Shearzyme 500L, Novozymes, Bagsvaerd, Denmark) at 125 units per g AXOS-15-0.27. After inactivation of the enzyme by boiling (30 min), the solution was lyophilised and the obtained material was homogenised and sieved

through a 250 $\mu$m sieve.

*Preparation of AXOS with avDP* of 39 *and A/X ratio of 0.22 (AXOS-39-0.22).* AXOS-39-0.22 was prepared from wheat squeegee WU-AX that was isolated from Meritena 233 B-starch as described above. Squeegee WU-AX, which had an average A/X ratio of 0.63, was suspended at 3 g/l in 25 mM sodium acetate buffer (pH 4.7) and incubated under continuous stirring for 2 h at 30°C with endoxylanase XBS (Grindamyl H640, Danisco, Denmark) at 3.3 units per g squeegee WU-AX After centrifugation (10,000 g, 15 min, 18°C) the supernatant was boiled for 30 min, to inactivate the enzyme, while the residue was washed with the earlier mentioned sodium acetate buffer (1/4 of the initial volume). The wash water was inactivated (30 min, 100°C) and combined with the supernatant. After filtration, the obtained solution was lyophilised to yield AXOS with an $avDP_{GC}$ of 262 and an A/X ratio of 0.50. The enzyme-solubilised material was further dissolved in deionised water (1:20 w/v) and the pH of the solution brought to 2.8 by addition of HCl (1M). The solution was incubated for 24 h at 90°C to remove a large fraction of the $\alpha$-1,2- and $\alpha$-1,3-linked arabinose substituents, which appear to be more prone to acid hydrolysis than the $\beta$-1,4-linked xylose subunits of AXOS. After cooling to room temperature, the solution was neutralised by addition of 1 M NaOH and centrifuged (10,000 g, 20 min, 18°C). Ethanol (95% v/v) was added to the obtained supernatant under continuous stirring to a final ethanol concentration of 80% (v/v). The mixture was stirred for an additional 30 min and kept overnight at 4°C. Precipitated AXOS compounds were recovered by centrifugation (10,000 g, 20 min, 4°C), dissolved in deionised water, lyophilised and the obtained material was homogenised and sieved through a 250 $\mu$m sieve.

*Preparation of AXOS with avDP* of 13 *and A/X ratio of 0.21 (AXOS-13-0.21).* AXOS-13-0.21 was prepared by incubating a solution of AXOS-39-0.22 (3 g/l) at 30°C during 2 h with XAA, an endoxylanase from *Aspergillus aculeatus* (Shearzyme 500L, Novozymes, Bagsvaerd, Denmark), at 27.7 units per g AXOS-39-0.22. After inactivation of the enzyme by boiling (30 min), the solution was lyophilised and the obtained material was homogenised and sieved through a 250 $\mu$m sieve.

*Characterisation of the isolated preparations.*

Different techniques were used to characterise the oligosaccharide preparations.

The total and reducing end sugar content was determined by gas-liquid chromatographic analysis as described by Courtin et al. (2000). The arabinoxylan (AX) content of samples was expressed as 0.88 x (% arabinose + % xylose). The average degree of polymerisation of AXOS as determined by gas-liquid chromatography ($avDP_{GC}$) was calculated as the sum of the total xylose and arabinose content divided by the reducing end xylose content. The protein content ($N \times 5.7$) of samples was determined by the Dumas combustion method, an adaptation of the AOAC Official Method (1995).

Profiling of the constituting oligosaccharides in the preparations was done by High Performance Anion Exchange Chromatography with Pulsed Amperometric Detection (HPAEC-PAD) using a Dionex DX-500 chromatographic system (Sunnyvale, CA, USA) equipped with an ED-40 electrochemical detector, a GP-50 gradient pump and an AS-3500 autosampler. Samples (10 mg) were solubilised in purified deionised water (2 ml, specific resistance 18 m$\Omega$-cm), filtered and injected (25 $\mu$l) on a Carbopac PA-100 guard column ($4 \times 25$ mm) attached to a Carbopac PA-100 anion exchange column ($4 \times 250$ mm). Elution (1.0 ml/min) was with a linear gradient of 0 to 250 mM sodium acetate in 100 mM NaOH during 30 minutes, followed by a linear gradient of 250 to 400 mM sodium acetate in 100 mM NaOH during 15 minutes. After the gradient, the column was eluted during 5 minutes with 100 mM NaOH. The elution was monitored using the ED-40 detector in the pulsed amperometric detection mode with following settings for potentials and time periods: $E_1$, + 0,05 V ($t_1$ = 400 ms); $E_2$, + 0,75 V ($t_2$ = 200 ms); $E_3$, - 0,15 V ($t_3$ = 400 ms). Arabinose (A), xylose ($X_1$), xylobiose ($X_2$) and XOS with DP of 3 to 6 ($X_3$ to $X_6$) were used as references.

High Performance Size Exclusion Chromatography (HPSEC) was performed on a HPLC system (Kontron Instruments, 325 pump systems, Kontron, Milan, Italy) equipped with autoinjection. All preparations were dissolved in 0.3% sodium chloride (1.5 ml), filtered and injected (20 $\mu$l) on a Shodex SB-800P guard column (Showa, Denko K.K., Tokyo, Japan, 50 $\times$ 6 mm) attached to a Shodex SB-806 HQ HPSEC column (300 x 8 mm, separation range: 1 x $10^2$ - 20 x $10^6$ Da). Elution was with 0.3% sodium chloride (0.5 ml/min; 30°C) and monitored with a refractive index detector (VDS Optilab, Berlin, Germany). Molecular mass markers were Shodex standard P-82 pullulans (2.0 mg/ml) with a molecular mass of 78.8 $\times$ $10^4$, 40.4 $\times$ $10^4$, 21.2 $\times$ $10^4$, 11.2 $\times$ $10^4$, 4.73 $\times$ $10^4$, 2.28 $\times$ $10^4$, 1.18 $\times$ $10^4$ and 0.59 $\times$ $10^4$ Da, and glucose with a molecular mass of 180 Da. The average degree of polymerisation (avDP) of oligosaccharides as determined by High Performance Size Exclusion Chromatography ($avDP_{HPSEC}$) was calculated as $MM_{HPSE}/132$.

*Determination of the activity of the xylanolytic enzymes.* The activity of the xylanolytic enzymes was measured colorimetric using xylazyme (Megazyme, Bray, Ireland) as an insoluble substrate according to manufacturer's instructions for the assay. One unit was defined as the amount of enzyme required to yield a change in extinction at 590 nm of 1.0 under the assay conditions.

Results and discussion

Different types of AXOS were prepared from either wheat bran, wheat flour or wheat squeegee starch as described in the Materials and Methods. The properties of the different preparations in terms of arabinoxylan content, average degree of substitution (arabinose to xylose ratio), and average degree of polymerisation are shown in Table 1, where they are compared to those of Xylooligo-95P, a commercial XOS preparation with known prebiotic properties (Campbell et al., 1997; Hsu et al., 2004). According to the nomenclature used in Table 1 and also hereinafter, AXOS preparations are referred to as AXOS-x-y, whereby x is the average degree of polymerisation as determined by gas-liquid chromatography and y is the ratio of arabinose to xylose. The different preparations had average degrees of polymerisation ranging from 7 to 122 (compared to 2 for Xylooligo-95P), and the degrees of substitution ranged from 0.21 to 0.78 (compared to 0.09 for Xylooligo-95P). The preparations derived from wheat bran (AXOS-7-0.34, AXOS-15-0.27, AXOS-8-0.27) had a lower degree of substitution than the preparations derived from wheat flour (AXOS-122-0.66, AXOS-16-0.78).

We have also shown that the degree of substitution of AXOS compounds can be reduced by acid treatment. Indeed, AXOS-39-0.22 is prepared from squeegee WU-AX by different steps including acid treatment (see Materials and Methods) and this reduces the average degree of substitution from 0.63 in squeegee WU-AX to 0.22 in AXOS-39-0.22.

Figure 2 shows the molecular mass distribution of the different AXOS preparations as determined by HPSEC. All AXOS preparations were polydisperse and consisted of molecular entities with a peak situated close to the avDP as determined by gas-liquid chromatography. The range of degrees of polymerisation within which 90% of the oligosaccharides fall, as determined from the HPSEC elution profiles, is provided in Table 1 for the different AXOS preparations.

**EXAMPLE 2: Physicochemical and sensory properties of XOS/AXOS preparations**

Materials and methods

*Oligosaccharide preparations.* Xylooligo-95P, AXOS-15-0.27, AXOS-39-0.22, and AXOS-13-0.21 were obtained as described in the Materials and Methods of example 1. The Fructo-oligosaccharide (FOS) preparation was the commercial product Raftilose (Orafti, Tienen, Belgium). The AXOS-15-0.27 used for sensory analysis was first dissolved in water (1:25 w/v) and treated with active carbon to remove possible off-flavours resulting from the production process. The suspension of AXOS-15-0.27 and active carbon (0.75 g/g AXOS-15-0.27) was stirred for 1 h at 18°C, and after decantation, the active carbon was removed by centrifugation (10000 g, 30 min, 18°C).

*Stability measurements.* Stability measurements were carried out on the water-extractable part of oligosaccharide preparations, which was obtained by suspension of the preparations in deionised water (1:10 w/v) followed by shaking (2 h, 18°C), centrifugation (10,000 g, 20 min, 18°C) and filtration. After lyophilisation of the filtrate, water-extractable samples were dissolved in an universal buffer with pH values of 2, 3, 7 and 11 to obtain solutions having oligosaccharide concentrations of 0.15% (w/v). The universal buffer was prepared from a stock solution of 6.0 g citric acid, 3.9 g potassium dihydrogen phosphate, 1.8 g boric acid and 5.8 g diethylbarbituric acid in water (1.0 l) (Britton and Welford, 1937). Aliquots (20 ml) of this stock solution were either adjusted with 2.0 M HCl or 2.0 M NaOH to obtain the desired pH.

Each of the oligosaccharide solutions was kept in boiling water for different time periods (0, 5, 10, 15, 20, 30 and 60 min). Thereafter, the solution was cooled and total monosaccharide and the reducing end sugar content were measured by gas-liquid chromatography as described in the Materials and Methods of example 1. The percentage degradation of FOS was calculated with formula (1), whereas the percentage degradation of Xylooligo-95P and AXOS-15-0.27 were calculated with formula (2). The percentage hydrolysis of FOS and of Xylooligo-95P and

AXOS-15-0.27 were calculated with formulae (3) and (4), respectively.

$$(1)\ \%\ \text{degradation} = \frac{(\text{total glucose + mannose conc. at time 0 and pH7}) - (\text{total glucose + mannose conc.})}{(\text{total glucose + mannose conc at time 0 and pH7})}$$

$$(2)\ \%\ \text{degradation} = \frac{(\text{total xylose + arabinose conc. at time 0 and pH7}) - (\text{total xylose + arabinose conc.})}{(\text{total xylose + arabinose conc at time 0 and pH7})}$$

$$(3)\ \%\ \text{hydrolysis} = \frac{(\text{reducing glucose + mannose conc.}) - (\text{reducing glucose + mannose conc. at time 0 and pH7})}{(\text{maximum amount of reducing glucose + mannose that can go in solution})}$$

$$(4)\ \%\ \text{hydrolysis} = \frac{(\text{reducing xylose + arabinose conc.}) - (\text{reducing xylose + arabinose conc. at time 0 and pH7})}{(\text{maximum amount of reducing xylose + arabinose that can go in solution})}$$

The percentage of hydrolysed xylose and arabinose linkages in AXOS-15-0.27 was calculated by formulae (5) and (6), respectively.

$$(5)\ \%\ \text{xylose hydrolysis} = \frac{(\text{reducing xylose conc.}) - (\text{reducing xylose conc. at time 0 and pH7})}{(\text{maximum amount of reducing xylose that can go in solution})}$$

$$(6)\ \%\ \text{arabinose hydrolysis} = \frac{(\text{reducing arabinose conc.}) - (\text{reducing arabinose conc. at time 0 and pH7})}{(\text{maximum amount of reducing arabinose that can go in solution})}$$

*Sensory analyses.* Sensory analyses were conducted in a quiet room in sessions involving maximally 10 volunteers at once. The subjects were asked to refrain from eating and drinking for at least 1 h prior to the session. The subjects were first familiarised with the procedures and subsequently asked to taste coded samples with different concentrations of Xylooligo-95P, AXOS-15-0.27, sucrose (reference sweet stimulus), sodium chloride (reference salty stimulus), and ascorbic acid (reference sour stimulus) prepared in purified deionised water (specific resistance 18 mΩ-cm). The solution with the highest concentration of each compound was numbered 8 and the lowest concentration was numbered 1 (Table 2). The subjects were asked to taste consecutively the full range of concentrations of one compound in order of increasing concentrations, yet the order by which the different compounds were presented to each individual was random. Before tasting the concentration series of a compound, the mouth was first rinsed twice with purified deionised water (specific resistance 18 mΩ-cm). and then 5 ml of each test solution was brought in the subject's mouth by means of a disposable syringe, swirled around for 5 seconds and then swallowed. The participants were asked to indicate the lowest concentration at which the taste of the compound could be recognized as sweet, salty or sour (individual recognition threshold). The average taste recognition threshold, determined as the concentration at which half of the subjects recognised the correct taste quality of a particular compound, was calculated with data on individual recognition thresholds obtained from 20 persons in total.

*Viscosity measurements.* Viscosity measurements were carried out on the water-extractable part of oligosaccharide preparations, which was obtained by suspension of the preparations in water (1:10 w/v) followed by shaking (2 h, 18°C), centrifugation (10,000 g, 20 min, 18°C) and filtration. After lyophilisation of the filtrate, the viscosity of a 5.0% (w/v) solution was measured with an Ostwald type viscosimeter (Capillary Viscosimeter, Schott Gerate, Type 50904) at 30°C according to Vinkx et al. (1991). Solution viscosity measurements were done in triplicates and expressed relative ($\eta_{rel}$) to that of deionised water by dividing the flow times of each of the oligosaccharide solutions by the flow times of deionised water under the same experimental conditions. After calculation of the specific viscosity ($\eta_{sp} = \eta_{rel} - 1$), the apparent intrinsic viscosity ($[\eta_{app}]$, dl/g) was determined using the Morris equation (Morris, 1984) as indicated in formula (7), where c (expressed as mg/ml) represents the oligosaccharide concentration, assuming that only oligosaccharides contribute to the viscous properties of the solutions. After each analysis, the viscosimeter was rinsed twice with deionised water, twice with 95% ethanol, once with acetone and once with diethyl ether and subsequently dried with compressed air.

$$(7)\ [\eta_{app}] = \frac{1}{c} \times \left[2\ [\eta_{sp} - \ln(\eta_{rel})]\right]^{0.5} \times 1000$$

## Results and discussion

The pH stability of AXOS-15-0.27 was compared to that of established prebiotic compounds, fnrcto-oligosaccharides (FOS, commercial product Raftilose) and xylo-oligosaccharides (XOS, commercial product Xylooligo-95P). The different oligosaccha-rides were kept at different pH values. (pH 2, 3, 7 and 11) at 100°C for different time periods. The percentage degradation was determined by measuring the decrease in content of the constituent monosaccharides, and the percentage hydrolysis was determined by measuring the increase in amount of reducing constituent monosaccharides. As shown in Figure 3, none of the oligosaccharides underwent substantial degradation at low or neutral pH (pH 2, 3 or 7). On the other hand, degradation was observed at alkaline pH (pH. 11) for all three oligosaccharides tested. This effect was most outspoken for Xylooligo-95P, of which 73% was degraded after 60 minutes. The most alkaline-stable oligosaccharide was AXOS-15-0.27 (Figure 3). The observed degradation at alkaline pH is known as alkali peeling, a reaction that occurs at elevated temperatures (60-100 °C) and progresses from the reducing end of the oligosaccharide (Whistler and BeMiller, 1958; Santori et al., 2003).

Of the three oligosaccharides tested, FOS was clearly the most sensitive to hydrolysis at low pH (Figure 4). At pH 2 and 3, 58% and 53% of FOS was hydrolysed after 60 minutes. Xylooligo-95P and AXOS-15-0.27 showed an equal level of hydrolysis at pH 2. However, AXOS-15-0.27 was more stable at pH 3 than Xylooligo-95P, with only 2% hydrolysis for AXOS-15-0.27 versus 6% for Xylooligo-95P (Figure 4). Further investigation of the hydrolysis of AXOS-15-0.27 at low pH revealed that the arabinose linkages are more susceptible to acidic hydrolysis than the xylose linkages. Indeed, incubation of AXOS-15-0.27 at pH 2, 100°C for 60 min caused hydrolysis of 54% of all arabinose linkages, whereas only 4% of the xylose linkages were hydrolyzed under these conditions (Figure 5).

Xylooligosaccharides with low DP such as Xylooligo-95P have been reported to taste sweet, with a sweetness being about 40% that of sucrose (Suntory, xylo-oligosaccharide, brochure and product sheet, 2001). Nothing is known so far about the taste properties of arabinoxylo-oligosaccharides with a larger degree of polymerisation. With a taste panel consisting of 20 persons we have determined the taste recognition threshold of AXOS-15-0.27 and Xylooligo-95P. The test samples also included sucrose, sodium chloride and ascorbic acid as representatives of sweet, salty and sour taste, respectively. The subjects were asked to indicate the concentration at which they recognised a taste as being sweet, salty or sour, for the concentration series of each of the test compounds. Only 15% of the taste panel noted a sweet taste for AXOS-15-0.27 at the highest concentration tested (71.5 g/l), and none of the subjects indicated a salty or sour taste. The average taste recognition threshold for sweetness of sucrose and Xylooligo-95P was 7 and 24 g/L, respectively, whereas that of AXOS-15-0.27 is higher than 71.5 g/L. AXOS-15-0.27 is therefore more than 10 times less sweet than sucrose and more than 3-fold less sweet than Xylooligo-95P, which by itself is about three times less sweet than sucrose.

The viscosity was determined for AXOS-15-27, AXOS-39-0.22 and AXOS-13-0.21, and compared to that of fructo-oligosaccharides (FOS, commercial product Raftilose) and xylo-oligosaccharides (XOS, commercial product Xylooligo-95P). As shown in Table 3, the different AXOS preparations have a higher apparent intrinsic viscosity that is 3 to 10 times higher relative to FOS and Xylooligo-95P.

## EXAMPLE 3: Fractionation of AXOS preparations by ultrafiltration

## Materials and methods

*Preparation of AXOS.* Squeegee WU-AX was prepared as described in the Materials and Methods of example 1. Squeegee WU-AX was suspended in sodium acetate buffer (25 mM, pH 4.7) at 3 g/l and incubated with XAA, an endoxylanase from *Aspergillus aculeatus* (Shearzyme 500L, Novozymes, Bagsvaerd, Denmark), at 18.4 U per g squeegee WU-AX for 4 h at 30°C. After inactivation of the enzymes by boiling for 30 min and subsequent filtration of the suspension, AXOS was recovered in the filtrate.

*Separation of AXOS by ultrafiltration.* AXOS were fractionated in a dead-end 'HP4750 stirred cell' ultrafiltration device (Sterlitech Corporation, Kent, USA). The ultrafiltration membranes used had a molecular mass cut off (MMCO) of either 5 kDa (P005F, Celgard, Wiesbaden, Germany), 10 kDa (PES-10, Synder Filtration, Vacaville, CA, USA), or 30 kDa (PES-030H, Celgard). Concentration polarization at the membrane surface was minimized by a Teflon-coated magnetic stir bar mechanism which was centrally positioned in the cell and had a stirring rate of 700 rpm. The pressure source was a compressed nitrogen gas cylinder. The pressure was controlled by a pressure regulator and all filtration experiments were carried out at a constant pressure of 4 bar at room temperature. The dead-end ultrafiltration cell was filled with 300 ml of the solution to be fractionated and filtration was stopped when a total volume of approximately 200 ml of permeate was collected.

*Characterisation of the isolated preparations.* See Materials and Methods in example 1. High Performance Size Exclusion Chromatography (HPSEC) was performed as described in the Materials and Methods in example 1, except that a Shodex SB-802.5 HQ HPSEC column (Showa, Denko K.K., Tokyo, Japan, 300 x 8 mm, separation range: $1 \times 10^2$ -$1 \times 10^4$ Da) was used. Molecular mass markers were Shodex standard P-82 pullulans (2.0 mg/ml) with a molecular mass of $11.2 \times 10^4$, $4.73 \times 10^4$, $2.28 \times 10^4$, $1.18 \times 10^4$ and $0.59 \times 10^3$ Da, xylo-oligosaccharide standards with a molecular mass of 810 (DP6), 678 (DP5), 546 (DP4), 414 (DP3) and 282 Da (DP2) and glucose with a molecular mass of 180 Da.

Results and discussion

The presence of sweet-tasting and metabolisable low molecular mass oligosaccharides in AXOS preparations can be undesired for particular applications as food component, whereas for other applications it is desired to use sweet-tasting AXOS preparations consisting mainly of low molecular mass oligosaccharides. For this reason it is useful to develop a method that allows fractionating AXOS preparations such that AXOS/XOS with DP2-3 are separated from AXOS with a higher DP. Fractionation of arabinoxylans has previously always been carried out by precipitation with alcoholic solutions of different concentrations (Courtin and Delcour, 1998). However, applying alcohol precipitation at an industrial scale is technically and economically unattractive. Therefore, the feasibility to further fractionate enzymically produced AXOS by ultrafiltration was investigated.

As an example, AXOS prepared by incubation of squeegee WU-AX with XAA (18.4 U/g) for 4 h at 30°C was subjected to ultrafiltration using membranes with a molecular mass cut-off (MMCO) of either 5 kDa, 10 kDa, or 30 kDa.

When the 5 kDa membrane is used, the HPSEC profile of the permeate fraction ($PER_{5kDa}$) (Figure 7a) mainly shows oligosaccharides corresponding in size to xylobiose (DP2) and xylotriose (DP3). The retentate fraction ($RET_{5kDa}$), which contains 86% of the soluble arabinoxylan in the AXOS stock solution (Table 4), shows basically the same molecular mass profile as the AXOS preparation before ultrafiltration, except that the amount of oligosaccharides with DP2 and DP3 is reduced by about half (Figure 7a). The incomplete removal of oligosaccharides with low DP in the $RET_{5kDa}$ fraction might result from their retention as a result of reduced permeate fluxes caused by the presence of high molecular mass components.

Through ultrafiltration using the 10 kDa membrane, 34% of the arabinoxylan content was recovered in the permeate fraction, while 65% was retained in the permeate fraction (Table 4). The permeate fraction ($PER_{10kDa}$) contained mainly oligosaccharides with DP2 to DP6.

The retentate fraction ($RET_{10kDa}$) had a low level of small-sized oligosaccharides with DP 2-4, and consisted mainly of oligosaccharides with a molecular mass ranging from 700 to >110,000 Da (DP5 to DP>850) (Figure 7b). Compared to the $PER_{10kDa}$ fraction, the permeate obtained with the 30 kDa membrane ($PER_{30kDa}$) had a higher content in oligosaccharides in the 1000 to 10,000 Da range. Correspondingly the retentate fraction of the 30 kDa membrane ($RET_{30kDa}$) had a lower content of oligosaccharides in the 1000 to 10,000 Da range in comparison to $RET_{10kDa}$, yet surprisingly the content of DP2 and DP3 oligosaccharides was higher in $RET_{30kDa}$ than in $RET_{10kDa}$.

From Table 4 it is clear that ultrafiltration influences the A/X ratio of the obtained AXOS fractions. The permeate fractions ($PER_{5kDa}$, $PER_{10kDa}$ and $PER_{30kDa}$) were enriched for oligosaccharides with a relatively low A/X ratio, while the retentate fractions ($RET_{5kDa}$,

$RET_{10kDa}$ and $RET_{30kDa}$) contained components with a higher A/X ratio.

To assess the possibility of further fractionation of AXOS by ultrafiltration, the $RET_{10kDa}$ fraction was subjected to a second ultrafiltration process in which a membrane with MMCO of 30kDa was used. The HPSEC profiles of the permeate fraction ($PER_{10kDa+30kDa}$) and retentate fraction ($RET_{10kDa+30kDa}$) obtained after ultrafiltration of the $RET_{10kDa}$ fraction through a membrane with MMCO of 30 kDa are, together with the $PER_{10kDa}$ fraction, shown in figure 8.

The $RET_{10kDa+30kDa}$ fraction contains mainly high molecular mass components (> 20,000 Da) which appear in the void volume of the Shodex SB-802.5 HQ column.. The $PER_{10kDa+30kDa}$ fraction, which represents approximately 25% of the arabinoxylans in the AXOS stock solution (Table 5), consists mainly of medium-sized oligosaccharides with a molecular mass ranging from 700 to 10,000 Da (DP 5 to 75) (figure 8).

These results indicate that ultrafiltration, preferably using a membrane with a MMCO of 10 kDa can be used as a method to make AXOS preparations with either a high content of oligosaccharides with DP2-6, by taking the permeate of the ultrafiltration, or a high content of oligosaccharides with DP>4 and a low content of DP2-4, by taking the retentate of the ultrafiltration. Consecutive ultrafiltration, preferably using the retentate of a 10 kDa membrane and passing that over a 30 kDa membrane, can be used to obtain an AXOS fraction that is enriched in AXOS ranging from DP 5-75.

**EXAMPLE 4: Effect of AXOS preparations on chicken intestines**

Materials and methods

*Oligosaccharide preparations.* See Materials and Methods in example 1.

*Microbiological analyses by plate counting.* The caecal content of chickens was weighed, diluted 1:10 in sterile peptone physiological salt solution (PPSS; 0.1% bacto peptone, 0.85% NaCl), homogenized in a stomacher, and further diluted in PPSS according to a tenfold dilution scheme. Appropriate dilutions were then plated using the pour plate technique on Violet Red Bile Glucose agar (VRBG; Oxoid CM0485B, Basingstoke, U.K) for the Enterobacteriaceae count, and on Wilkins-Chalgren anaerobe agar (Oxoid CM619) modified by addition of glacial acetic acid (1 ml/l) and mupirocin (100 mg/l) for the bifidobacteria. The latter modifications enhance the selectivity of Wilkins-Chalgren agar for bifidobacteria, and modified Wilkins-Chalgren agar showed particularly suitable for the one step determination of the bifidobacterial count in chicken caeca (Rada et al., 1999; Rada and Petr, 2000). Colonies were counted after 1 day of aerobic incubation at 37°C for Enterobacteriaceae, and after 4 days of anaerobic incubation (in anaerobic jars with Oxoid anaerogen system AN0025A) at 37°C for the bifidobacteria. Counts were expressed per g of caecal content. Several precautions were taken to minimize exposure of bifidobacteria to oxygen during sample preparation: (i) caecal content was only removed from the caeca just before the start of the sample preparation; (ii) diluents and agars were autoclaved or boiled just before use to remove dissolved oxygen; (iii) plates for bifidobacterial counts were placed under anaerobic atmosphere within 2 h from the start of sample preparation.

*Microbiological analyses by quantitative PCR.* Extraction of DNA was performed as described by Van de Wiele et al (2004) on caeca samples diluted 1:5 in PPSS. Amplification was performed in 25 $\mu$l reactions mixtures by using buffers supplied with the SYBR® Green PCR Core Reagents as described by the suppliers (PE Applied Biosystems, Nieuwerkerk a/d Ijssel, The Netherlands) in MicroAmp Optical 96-well reaction plates with optical caps (PE Applied Biosystems). Forward and reverse primers, BIF164f and BIF163r respectively (Satokari et al., 2001), were used at a concentration of 1 $\mu$M for detection of copies of the 16S ribosomal RNA genes from bacteria of the genus *Bifidobacterium.* The PCR temperature program was as follows: 50°C for 2 min, 95°C for 10 min, followed by 40 cycles of 94°C for 1 min, 62°C for 1 min, and 60°C for 1 min. The template DNA was amplified in triplicate reaction mixtures and monitored with an ABI Prism SDS7000 instrument (PE Applied Biosystems). Standard curves were constructed based on real-time PCR amplification in quadruplicate reactions on four different dilutions of DNA extracted from a culture of *Bifidobacterium breve* (strain LMG11042). Real-time PCR data from the experimental samples were plotted against the standard curve and corrected for efficiency of DNA extraction using a factor consisting of the DNA concentration in the experimental sample with the highest DNA concentration divided by the DNA concentration of each individual experimental sample.

*Statistical analyses.* The effect of different diets on body mass, feed intake or the microbial composition of the intestines of animals was analysed by 1-factor variance analysis at the 95% confidence level using the Analyse-it software, version 1.71. In case a statistically significant effect was observed for the factor diet, differences between the diets were analysed either by the least significant difference (LSD) test or by Tukey's test at the 95% confidence level.

Results and discussion

The effect of the addition of different (arabino)xylan derived preparations was tested on the microbial composition of the lower intestines of chickens.

For a first experiment, 64 one-day-old male chickens (Ross roosters) were purchased from a commercial hatchery (Avibel, Tielt, Belgium) and distributed over 4 pens (16 chickens per pen). In each pen a drinking basin and a feed container of 1 m was present. The temperature of the stable was 35°C at the arrival of the birds and was subsequently decreased with 1 ° C every 2 days. The animals were kept at a photoperiod of 23 hours light and 1 hour darkness.

The feeds (1 feed per pen) and drinking water were administered *ad libitum.* The duration of the experiment was 14 days.

The following experimental feeds were tested:

- Control feed
- Control feed + 0.25% AXOS-7-0.34 preparation (0.17% pure AX)
- Control feed + 0.25% AXOS-122-0.66 preparation (0.18% pure AX)
- Control feed + 0.25% Xylooligo-95P (0.22% pure AX)

The concentrations indicated between brackets were corrected for their purity as calculated by their AX content. The control feed consisted of a commercial starter feed (Krix 0, Hendrix UTD, Boxmeer, The Netherlands) that contained a xylanase and glucanase enzyme mix (Roxazyme) as an additive.

At the age of seven days all animals were weighed and 8 animals per group were randomly selected and sacrificed by decapitation. Thereafter, the animals were dissected to collect the caeca. Caeca were emptied and the contents were pooled in two groups per treatment (four caeca each). This protocol was repeated when the remaining animals reached the age of fourteen days, except that the caeca were pooled in three groups per treatment (from three, three, and two animals each).

For the animals in the AXOS-7-0.34 group, no significant differences in mean body weight were observed relative to the control group. The animals in the AXOS-122-0.66 and

Xylooligo-95P groups had a significantly lower body weight than the control group after seven days, but the animals caught up after 14 days and the difference with the control became non-significant. In the case of the AXOS-122-0.66 group, the relatively lower body weight after 7 days might have been due to a significantly lower weight of the chickens in this group at hatching.

Analysis of the caecal microbiota of the chickens indicated high levels of enterobacteriaceae (about $10^8$-$10^9$/g caecal content), both after 7 days and 14 days of feeding, and no significant differences were observed for any of the treatments. The levels of bifidobacteria after 7 days were low in all treatment groups (about $10^2$-$10^3$/g caecal content) (Figure 9), except for the animals in the Xylooligo-95P group for which the level of bifidobacteria was much higher (about $10^8$/g caecal content). After 14 days the number of bifidobacteria in the caecum stayed low for the control group. In contrast, a clear increase in the number of bifidobacteria (by a factor of about $10^5$) was observed in the caecal content of the AXOS-7-0.34 group. In the Xylooligo-95P group, the level of bifidobacteria was somewhat lower relative to the level after 7 days, yet after 14 days it was still significantly higher than for the control group. In the

AXOS-122-0.66 group a moderate increase of the number of bifidobacteria in the caecal content occurred after 14 days, however this increase was not significant relative to the control group.

For a second experiment on chickens, 54 one-day-old male chickens (Ross roosters) were purchased from a commercial hatchery (Avibel, Tielt, Belgium) and distributed over 6 pens (9 chickens per pen). Pen conditions were as described for the first experiment (see above).

The duration of the experiment was 14 days.

Following experimental feeds were tested:

- ■ Control feed
- ■ Control feed + 0.141% AXOS-15-0.27 preparation (0.1% pure AX)
- ■ Control feed + 0.352% AXOS-15-0.27 preparation (0.25% pure AX)
- ■ Control feed + 0.263% FOS preparation (0.25% pure FOS)
- ■ Control feed + 1.053% FOS preparation (1% pure FOS)
- ■ Control feed + 0.01 % Xylanase preparation

The concentrations indicated between brackets were corrected for their purity as calculated by their AX or FOS content. The FOS preparation was the commercial product Raftilose (Orafti, Tienen, Belgium). The xylanase preparation was the commercial product Belfeed (Beldem, Groot-Bijgaarden, Belgium). The composition of the control feed is given in Table 6. At the age of 14 days all animals were weighed and sacrificed by decapitation. Thereafter, the animals were dissected to collect the caeca, and the contents of the caeca were pooled per 3 animals belonging to the same treatment group. The number of bifidobacteria in the pooled caecum samples was measured by quantitative PCR.

After two weeks of feeding the respective diets, no significant differences in body weight could be observed between the different treatments. With respect to the number of bifidobacteria in the caecum of the chickens after 2 weeks, only the chickens fed the diet containing 0.25% AXOS-15-0.27 differed significantly from the control treatment (Figure 10).

The number of bifidobacteria in chickens fed with 0.25% AXOS-15-0.27 was 22-fold higher than in the chickens fed the control diet. It should be noted that fructo-oligosaccharides (FOS), a well documented prebiotic compound which was included for comparative purposes, did not cause an increase in the number of bifidobacteria in chicken caeca, even at a dose 4 times higher than that at which AXOS-15-0.27 is effective. This indicates that

AXOS-15-0.27 is a surprisingly potent bifidogenic compound.

Bifidobacteria have been positively associated with animal and human health and are components of many probiotic compounds. There has been an increasing interest in selectively enriching these bacterial populations within the gastro-intestinal tract, in addition to directly feeding microbial preparations, to promote or maintain a positive health status in animals and humans, including a suppressive effect on colorectal cancer (Van Loo et al., 2004). The results of the feeding trials with chickens indicate that all arabinoxylan containing feeds stimulated the presence of bifidobacteria in the caeca of the chickens. The AXOS preparation with high degree of polymerisation, AXOS-122-0.66, caused only a moderate non-significant increase in bifidobacterial population in the caecum of chickens. On the other hand, the preparations with low to intermediate degree of polymerisation, Xylooligo-95P, AXOS-7-0.34 and AXOS-15-0.27, triggered a strong and significant increase in bifidobacteria without causing a reduction of the body weight of the animals. The response to Xylooligo-95P could be observed already after 7 days, whereas AXOS-7-0.34 and AXOS-15-0.27 only caused a bifidogenic response after 14 days. This indicates that Xylooligo-95P is fermented faster by Bifidobacteria than the AXOS-7-0.34 and AXOS-15-0.27 preparations which have a higher average degree of polymerisation and a higher degree average degree of substitution.

Slow fermentation by selected beneficial bacteria is a desired property of a prebiotic compound for application in humans and other animals with long bowels. Slow fermentation by intestinal bacteria increases the fraction of the prebiotic compound that is not fully consumed after passage through the proximal parts of the colon and thus can be used as a substrate for fermentation by the appropriate bacteria in the distal parts of the colon. In humans the incidence of cancer in the distal and rectal parts of the colon, taken together, are higher than that of the proximal parts of the colon (http://www.state.nj.us/health/cancer/cr/subsites.htm), and it is therefore important that prebiotic compounds can stimulate the beneficial microbiota of the distal colon and rectum.

## EXAMPLE 5: Effect of AXOS preparations on rat intestines

### Materials and methods

*XOS/AXOS preparations*. See Materials and Methods in example 1.

*Microbiological analyses by quantitative PCR.* See Materials and Methods in example 4.

*Short chain fatty acid analysis*. Intestinal samples were extracted with 5 volumes of water/phosphoric acid/ formic acid (98/1.8/0.2, v/v/v) and clarified by centrifugation (22,000 g, 10 min). Short chain fatty acids (SCFAs) were analysed by gas-liquid chromatography (Shimadzu, GC 14A) on a column packed with Chromosorb W (mesh size 60/30; Supelco, Bellefonte, USA) and detected by flame ionization. Concentrations of SCFAs were calculated based on standards with known concentrations of the different acids. Capronic acid was used as an internal standard.

*Statistical analyses*. The effect of different diets on body mass, feed intake, SCFA or the microbial composition of the intestines of animals was analysed by 1-factor variance analysis at the 95% confidence level using the Analyse-it software, version 1.71. In case a statistically significant effect was observed for the factor diet, differences between the diets were analysed by the least significant difference (LSD) test at the 95% confidence level.

### Results and discussion

For a first experiment on rats, 40 6-week-old male rats (Wistar) were purchased from Elevage Janvier (Le Genest-St-Isle, France) and housed in stainless steel wire-bottom cages (2 rats per cage) in an environmentally controlled room (22°C) with a 14-10 h light-dark cycle. Rats were given free access to water and to pellets (10 mm) of a 'basic humanised diet' during 7 days. The composition of the 'basic humanised diet' is given in Table 7. After 7 days of adaptation on the basic humanised diet, the rats were randomly assigned to one of 5 different treatment groups (8 rats/group), and the groups were each given free access to pellets (10 mm) of one of the following diets during 13 days:

- ■ Basic humanised diet
- ■ Basic humanised diet + 0.70% AXOS-122-0.66 preparation (0.5% pure AX)
- ■ Basic humanised diet + 0.69% AXOS-16-0.78 preparation (0.5% pure AX)
- ■ Basic humanised diet + 0.73% AXOS-15-0.27 preparation (0.5% pure AX)
- ■ Basic humanised diet + 0.72% AXOS-8-0.27 preparation (0.5% pure AX)

The concentrations indicated between brackets were corrected for their purity as calculated by their AX content. For the AXOS containing diets, the starch in the basic humanised diet was replaced with the appropriate amount of AXOS.

Animals were weighed and feed intake was measured 3 times per week. After 13 days of treatment, all animals were weighed and euthanized with an overdose of Nembutal. Thereafter, the animals were dissected to collect the faeces. The faeces were collected as pellets from the distal colon to the anus. The faeces were pooled per 2 animals belonging to the same treatment group and analyzed for SCFA content.

Feed intake during the treatment period ranged from 18.9 to 27.7 g/day with an average of 22.7 g/d. Initial body weights of the rats at the start of the treatment were on average 262.0 g, and final body weights after 13 days of treatment were on average 375.3 g. No significant differences in body weight or feed intake could be observed between the different treatments.

As increased intestinal SCFA levels are the hallmark of shifts in the intestinal microflora induced by intake of prebiotic compounds (Van Loo, 2004), we have measured the SCFA content of faeces for the different treatment groups. As shown in Figure 11, the propionate level in the faeces of rats fed AXOS-8-0.27, AXOS-15-0.27 and AXOS-16-0.78 were significantly higher than that in the control group or the AXOS-122-0.66 group. There was also a trend for increased levels of acetate and butyrate in the faeces of the AXOS-8-0.27, AXOS-15-0.27 and AXOS-16-0.78 groups.

SCFAs such as acetate, propionate and butyrate are produced as electron sinks of respiration by bacteria in the anaerobic environment of the gut. Prebiotic compounds are known to increase SFCA levels in the lower gastro-intestinal tract. High levels of SCFAs are desirable because they reduce the pH of the gut content and thereby limit the growth of potentially pathogenic putrefactive bacteria. The lowering of the intestinal pH also entails an increase in the solubility and bio-availability of the minerals calcium and magnesium (Teitelbaum and Walker, 2002). Short chain fatty acids, especially butyrate, moreover stimulate colon epithelial cells, thereby increasing the absorptive capacity of the epithelium (Topping and Clifton, 2001).

The fact that no significant differences were observed among the rats treated with AXOS-15-0.27 or AXOS-16-0.78, two preparations with approximately the same average degree of polymerisation but with a different A/X ratio, indicates that the degree of substitution does not have a large impact on the ability of AXOS to stimulate bacterial fermentation in the colon. On the other hand, when comparing the effects of AXOS-16-0.78 and AXOS-122-0.66, two preparations with approximately the same degree of substitution but with a different degree of polymerisation, it is clear that AXOS preparations with a high degree of polymerisation such as AXOS-122-0.66 are less potent prebiotic compounds than preparations with a lower average degree of polymerisation. Therefore, AXOS preparations with an average degree of polymerisation below 120 are preferred prebiotic compounds. In case slow fermentation is preferred, in order to increase availability of AXOS in the distal colon and rectum, AXOS preparations with an average degree of polymerisation ranging from above 4 are preferred.

For a second experiment on rats, 24 6-week-old male rats (Wistar) were purchased from Elevage Janvier (Le Genest-St-Isle, France) and housed in stainless steel wire-bottom cages (2 rats per cage) in an environmentally controlled room (22°C) with a 14-10 h light-dark cycle. Rats were given free access to water and to pellets (10 mm) of a 'basic humanised diet' during 6 days. The composition of the 'basic humanised diet' is given in Table 7. After 6 days on the basic humanised diet, the rats were randomly assigned to one of 3 different treatment groups (8 rats/group), and the groups were each given free access to pellets (10 mm) of one of the following diets:

- ■ Basic humanised diet
- ■ Basic humanised diet + 5.87 % AXOS-15-0.27 preparation (4 % pure AX)
- ■ Basic humanised diet + 5.26 % Xylooligo-95P (4 % pure AX)

The concentrations indicated between brackets were corrected for their purity as calculated by their AX content. For the diets containing AXOS-15-0.27 or Xylooligo-95P, the starch in the basic humanised diet was replaced with the appropriate amount of AXOS.

Animals were weighed and feed intake was measured 3 times per week. After 14 days of treatment, all animals were weighed, euthanized with an overdose of Nembutal, and dissected to collect the proximal colon, the caecum, and the distal colon. The contents of the caecum were pooled per 2 animals belonging to the same treatment group and analyzed for the amount of bifidobacteria by quantitative PCR. The contents of the proximal colon and the distal colon were pooled per 2 animals belonging to the same treatment group and analyzed for SCFA content.

In the proximal colon, SCFA levels were much lower than in the distal colon, and only acetate was above the detection threshold in proximal colon samples. Hence, data could only be obtained for acetate in the proximal colon and for acetate, propionate and butyrate in the distal colon. None of the treatments resulted in a significant effect on SCFA levels at a 95% probability level. However, clear trends could be observed from the data. As shown in Figure 12, the AXOS-15-0.27 and Xylooligo-95P groups had higher levels of acetate and butyrate, but not propionate, compared to the control group. The most striking effect was observed for butyrate in the distal colon, for which the increase relative to the control group was 58% in the AXOS-15-0.27 group and 34% in the Xylooligo-95P group. The stronger effect of AXOS-15-0.27 relative to Xylooligo-95P was observed for all SCFAs in the distal colon. Interestingly, Xylooligo-95P caused the strongest acetate increase in proximal colon, whereas AXOS-15-0.27 resulted in the strongest acetate boost in the distal colon.

The amount of bifidobacteria was significantly increased in the caeca of rats fed either AXOS-15-0.27 or Xylooligo-95P compared to rats that received the control diet. The highest increase in caecal bifidobacteria was observed in the AXOS-15-0.27 group. (1.08 log units or 12 times higher versus the control group, Figure 13).

These results indicate that AXOS-15-0.27 is a more potent prebiotic compound than Xylooligo-95P. The data also indicate that AXOS-15-0.27 is fermented more slowly than Xylooligo-95P since the Xylooligo-95P causes a stronger SCFA effect in the proximal colon while AXOS-15-0.27 caused a stronger effect in the distal colon. The slower fermentation of AXOS-15-0.27 versus Xylooligo-95P was also observed in the experiments on chickens presented in example 5. The slow fermentation properties of AXOS-15-0.27 are desired since it results in more prebiotic compound reaching the distal colon, where it can exert its beneficial and presumed carcinogenesis-suppressing effects.

**EXAMPLE 6: Effect of AXOS preparations on human intestines**

Materials and methods

*AXOS-15-0.27 and WPC preparations.* See Materials and Methods of example 1.

*Microbiological analyses by quantitative PCR.* See Materials and Methods in example 4.

*Subjects.* None of the volunteers participating in the experiments had a history of gastrointestinal or metabolic disease or previous surgery. The subjects were free of antibiotics or any other medical treatment influencing gut transit or intestinal flora for at least 3 months before the start of the study. None of the women were pregnant during the experiments. No standard diets were imposed on the volunteers. However, they were asked to maintain a regular eating pattern until the end of the study period and to avoid excessive intake of fermented milk products and food components containing high quantities of fermentable carbohydrates. The Ethical Committee of the University of Leuven approved the experiments and all subjects gave informed consent.

*Labelled substrates.* Lactose-[$^{15}$N,$^{15}$N]-ureide was synthesized according to the method of Schoorl as modified by Hofmann (1931) with [$^{15}$N,$^{15}$N]urea obtained from Euriso-top (Saint-Aubin, France). $^{3}$H-labelled polythyleneglycol ($^{3}$H-PEG) was obtained from New England Nuclear Life Science Products (Boston, MA, USA).

*Determination of urinary and fecal $N_{tot}$ and $^{15}$N.* Total N content and $^{15}$N enrichment of urine and faeces were measured by using an elemental analyzer (ANCA-SL; PDZ, Cheshire, UK) coupled with both a thermal conductor detector (TCD; PDZ) and a stable isotope ratio mass spectrometer (IRMS; PDZ). A known volume of urine (15 $\mu$l) or a known mass of faeces (about 5-7 mg of freeze-dried stool) was oxidized in the presence of oxygen at 1,000°C. The combustion products thereafter passed through a second furnace containing copper at 600°C where excess oxygen was absorbed and nitrogen oxides were reduced to elemental nitrogen. Total nitrogen content was measured by means of a TCD detector, whereas the $^{15}$N enrichment was determined by means of an IRMS detector, coupled to the combustion unit of the elemental analyzer. The $^{15}$N to $^{14}$N isotope ratio of $N_2$ was measured with reference to a calibrated laboratory standard (i.e. a standard ammonium sulfate solution). The percentage of administered dose of $^{15}$N recovered was calculated as follows (Evenepoel *et al.*, 1999):

$$\% \text{ dose } {}^{15}\text{N} = 100 \times \frac{\text{mg excess } {}^{15}\text{N}_t}{\text{mg } {}^{15}\text{N } administered}$$

where *mg excess*

$$^{15}N_t = \frac{AP_t - AP_{bas}}{100} \times N_{tot}$$

and

$AP_t$: the measured $^{15}$N enrichment of a specified urine sample, expressed in atom percent (AP)

$AP_{bas}$: the $^{15}$N enrichment of a basal urine sample (expressed in AP)

$N_{tot}$: the total nitrogen content in a specified urine sample

For urine samples, the percentage of administered $^{15}$N dose was cumulated for the period of 0-48h after the test meal, whereas for faecal samples the percentage of administered $^{15}$N dose was cumulated for the period of 0-72h after the test meal. A correction for interindividual oro-faecal transit time differences was performed for the faecal samples. This was done by dividing the cumulative percentage of administered dose $^{15}$N recovered over 72 h by the cumulative percentage of administered dose of $^{3}$H-PEG recovered over 72 h. The $^{3}$H-PEG content in stool was measured by liquid-scintillation counting (Tricarb liquid scintillation spectrometer, model 3375; Packard Instruments, Downers Grove, IL, USA) after oxidation to $^{3}$H-H$_2$O (Packard sample oxidizer, model 307).

*Determination of urinary phenolic compounds.* Total p-cresol and phenol content were measured by Gas-liquid Chromatography - Mass Spectrometry (GC-MS) technology. The pH of 950 ml urine was adjusted to pH 1 with concentrated H$_2$SO$_4$. This solution was heated for 30 min at 90°C to deproteinise and hydrolyse the conjugated phenols. After cooling down to room temperature, 50 ml 2,6-dimethylphenol (20 mg/100 ml) was added as an internal standard. The phenols were extracted with 1ml ethyl acetate. The ethyl acetate layer was dried with Na$_2$SO$_4$ and 0.5 ml of this solution was analysed on a GC-MS (Trace GC-MS, Thermo Finnigan, San José, CA, USA). The analytical column was a 30 m x 0.32 mm internal diameter, 1$\mu$m AT5-MS (Alltech Associates, Deerfield, IL, USA). Helium gas, GC grade, was used as a carrier at a constant flow rate of 1.3 ml/min. The oven temperature was programmed from 75°C (isothermal for 5 min), and increased by 10°C/min to 160°C and by 20°C/min to 280°C. Mass spectrometric detection was performed in electron impact full scan mode from m/z 59 to m/z 590 at two scans/s. Results for p-cresol and phenol were expressed as total content (mg) recovered in 0-24 h collections.

*Statistical analysis.* The statistical analysis was performed with SPSS software, version 12.0. Statistical evaluation of the data was performed by applying a Wilcoxon Signed Ranks test at the 95% confidence level.

Results and discussion

A first experiment on humans was performed on 10 healthy volunteers (5 men and 5 women, age range 23-37 years). The 10 volunteers were randomly allocated to either of two groups of 5 persons. The first group, received for 2 weeks per day 7 g (4.88 g) of AXOS-15-0.27 suspended in water, whereas the second group received daily 11.6 g (4.82 g) of WPC suspended in water. The weight values between brackets are corrected weights for AXOS on the basis of AX content and moisture content of the preparation.

For each of the volunteers faecal samples were collected during the day prior to the start of the experiment, and during the 15th day. Each time the first stool voiding of the day was collected, weighed, and stored at -20°C until microbial analysis by quantitative PCR.

As shown in Figure 14, the subjects receiving 4.88 g/day of AXOS-15-0.27 during 14 days had a level of faecal bifidobacteria that was 1.55 log units (= 35 times) higher relative to the basal level (p = 0.043). In contrast, the level of bifidobacteria was slightly reduced after 2 weeks in the subjects administered 4.82 g/day WPC, although the difference relative to the basal level was not significant (p=0.715).

These data indicate that AXOS compounds with an intermediate average degree of polymerisation such as AXOS-15-0.27 (avDP = 15, Table 1) exert a stronger prebiotic effect than compounds with a high average degree of polymerisation such as WPC (avDP = 58, Table 1). The same conclusion could already be drawn from tests on bifidogenic effects in chickens (see example 4) and on the increase of SCFAs in rats (see example 5).

In a second experiment the effect of different once-off doses of AXOS-15-0.27 was assessed on the metabolism of $NH_3$, p-cresol and phenol in the human colon. $NH_3$, phenol and p-cresol are toxic and potentially carcinogenic metabolites that are produced by bacteria in the colon upon fermentation of proteins. $NH_3$ metabolism was assessed using lactose-[15N,15N]-ureide as a biomarker. On oral administration, lactose-ureide reaches the colon unmodified, because the molecular bond between the carbohydrate moiety and urea in lactose-ureide resists enzymatic degradation in the human gastrointestinal tract. When lactose-[15N,15N]-ureide reaches the colon, it is degraded to [15N,15N]-labeled urea by selected bacteria. The labeled urea undergoes fast hydrolysis with production of [15]$NH_3$ (Wutzke et al. 1997). The labeled $NH_3$ is mixed with the ammonia present in the colon, and as a consequence, the variations observed in the $^{15}$N-labeled measurement reflect the fate of total colonic $NH_3$.

In addition to monitoring colonic $NH_3$ metabolism, the presence of phenol and p-cresol were measured in the urine as hallmarks of bacterial fermentation of proteins in the colon. Based on literature data (Evenepoel et al. 1999), it is assumed that in physiological circumstances about 3 to 6% of the proteins ingested remains undigested. When the non-digested proteins reach the colon, they are fermented by the colonic flora. Bacterial fermentation of the amino acid tyrosine results in the production of phenol and p-cresol. These compounds are largely absorbed from the colon, detoxified in the mucosa and in the liver (by glucuronide- and sulphate- conjugation) and finally excreted in the urine. Since phenol and p-cresol originate exclusively from bacterial metabolism and not from human metabolism, the urinary output of phenol and p-cresol reflects the bacterial production of these compounds in the colon (Smith and Macfarlane, 1996). As a consequence, any influence of prebiotic compounds on the colonic protein fermentation should be reflected in the urinary concentration of phenol and p-cresol.

The second experiment on humans was performed on 9 healthy volunteers (3 men and 6 women, age range 19-26 years). Each volunteer received 5 different test meals containing different doses of AXOS-15-0.27 with an interval of 1 week between each test meal. The different AXOS-15-0.27 doses per day were either 0, 0.35 g (0.24g), 1.05 g (0.73 g), 3.17 g (2.21 g) and 7 g (4.88 g), with the weight values between brackets being corrected weights for AXOS on the basis of AX content and moisture content of the preparation. The order by which the volunteers received the different test meals was random. The test meal consisted of a pancake (15,8 g proteins, 11,6 g fat and 21,1 g carbohydrates; 255 kcal) containing 75 mg of the stable isotope labelled substrate lactose-[15N,15N]-ureide, 185 kBq of tritium-labelled polyethylene glycol ($^3$H-PEG), and AXOS-15-0.27 at the appropriate dose. $^3$H-PEG was used as a biomarker for oro-faecal transit time (Geboes et al 2005).

Urine was collected in recipients to which 1 gram of neomycin was added for prevention of bacterial growth. A basal urine sample was collected before consumption of the test meal. After intake of each test meal, a 48 h urine collection was performed in 3 different fractions: 0-6 h, 6-24h and 24-48 h. After measurement of the urine volume, samples were taken and stored at -20°C until analysis. Stools were collected over 72 h after the test meal. The stools were frozen immediately after voiding, weighed, and then stored at -20°C. All stools collected on the same day were combined and homogenized before further analysis. Samples of known weight were removed and freeze-dried. The dried material was weighed again, and aliquots were taken for analysis of nitrogen and radioactivity.

As shown in Figure 15A, a consistently lower urinal excretion rate of $^{15}$N-labelled nitrogen was noted after intake of the test meal containing AXOS-15-0.27 as compared to the control meal lacking AXOS-15-0.27. 44.5 % of the administered $^{15}$N dose was retrieved in urine samples after consumption of the control meal. This fraction was lowered to 40.4% (-9% relative to control), 43.7% (-2% relative to control), 33.1% (-26% relative to control) and 33.6% (-25% relative to control) after ingestion of 0.24, 0.73, 2.21 and 4.88 g AXOS-15-0.27, respectively. The decrease in urinary $^{15}$N excretion was significant at $p < 0.05$ for the AXOS-15-0.27 doses of 2.21 and 4.88 g.

Conversely, AXOS-15-0.27 supplementation to the test meals caused a consistent increase in $^{15}$N excretion via the faeces (Figure 15 B). In the control test meal group, 15.1 % of the administered $^{15}$N dose was recovered in the faeces. This fraction was increased to 16.4% (+8% relative to control), 18.8% (+24% relative to control), 25.3% (+67% relative to control) and 26.1% (+72 % relative to control) after ingestion of 0.24, 0.73, 2.21 and 4.88 g, respectively. The increase in faecal $^{15}$N excretion was significant at $p < 0.05$ for the AXOS-15-0.27 doses of 2.21 and 4.88 g. AXOS-15-0.27 had no effect on the gastrointestinal transit time, as measured via the $^3$H-PEG biomarker, at any of the doses tested (Figure 15 C).

A consistently lower urinal excretion rate of p-cresol and phenol was noted after intake of test meals containing AXOS-15-0.27 as compared to the control meal lacking AXOS-15-0.27 (Figure 16). The fraction of p-cresol excreted via the urine was lowered by -9%, -21%, -35% and -41% relative to control, after ingestion of 0.24, 0.73, 2.21 and 4.88 g AXOS-15-0.27, respectively (Figure 16A). The fraction of phenol excreted via the urine was lowered by - 45%, -39%, -33% and -45% relative to control, after ingestion of 0.24, 0.73, 2.21 and 4.88 g AXOS-15-0.27, respectively (Figure 16B). The decrease in urinary phenol excretion was significant at $p < 0.05$ for all of the AXOS-15-0.27 doses tested, ranging from 0.24 g to 4.88 g. AXOS-15-0.27 has thus been demonstrated to be a prebiotic compound in humans that is active at surprisingly low doses, down to 0.24 g per serving.

Reduction of urinary excretion of nitrogen and concomitant increase of faecal N-excretion has previously been observed in humans that were administered known prebiotic compounds such as inulin and lactulose (De Preter et al. 2004; Geboes et al. 2005). Stimulated excretion of ammonia via the faeces is desired since ammonia has been shown to reduce the life span of colonic epithelial cells, to increase their turnover, and to make them more susceptible to chemical carcinogenesis (Visek 1978). Ammonia in the lumen of the colon is mainly derived from bacterial fermentation of non-digested proteins (Smith & Macfarlane, 1996; Fooks et al., 1999). Slowly fermentable prebiotic carbohydrates, such as AXOS with intermediate degrees of polymerisation, stimulate saccharolytic bacteria and saccharolytic pathways in bacteria present in the colon. Such compounds thus repress proteolytic fermentation in the colon and stimulate reutilisation of ammonia as a nitrogen source by the bacteria that rely on carbohydrates as main carbon and energy source. Along the same line, reduction of urinary excretion of the protein fermentation metabolites cresol and phenol is desired since these phenolic compounds have been implicated in bowel cancer (Bone et al. 1976). Reduced urinary excretion of phenolic compounds has previously been observed for the prebiotic compound lactulose (De Preter et al. 2004). It is assumed that decreased excretion of cresol and phenol are caused by an increased utilisation of the amino acid tyrosine or metabolic products of protein putrefaction through increased saccharolytic bacterial activity in the colon, as stimulated by prebiotic compounds.

**REFERENCES**

AOAC, 1995. Official Methods of Analysis, 16th edition. Method 990.03. Association of Official Analytical Chemists, Washington DC, USA.

Beaugrand J., Chambat G., Wong V.W.K., Goubet F., Rémond C., Paës G., Benamrouche S., Debeire P., O'Donohue M., Chabbert B. (2005) Impact and efficiency of GH10 and GH11 thermostable endoxylanases on wheat bran and alkali-extractable arabinoxylans. Carbohydr. Res. 339: 2529-2540

Biely, P., Vrsanska, M., Tenkanen, M., Kluepfel, D. (1997) J Biotechnol, 57: 151-166

Bone E, Tamm A and Hill M (1976) The production of urinary phenols by gut bacteria and their possible role in the causation of large bowel cancer. Am J Clin Nutr 29, 1448-1454.

Britton H.T.S, Welford, G. (1937) J. Chem. Soc. 1848.

Campbell J.M., Fahey, G.C., Wolf, B.W. Selected indigestible oligosaccharides affect large bowel mass, cecal and fecal short-chain fatty acids, pH and micorflora in rats (1997), J. Nutr. 127:130-136

Campbell, G.L.; Rossnagel, B.G.; Classen, H.L.; Thacker, P.A. 1989. Genotypic and environmental differences in extract viscosity of barley and their relationship to its nutritive value for broiler chickens. Animal Feed Science and Technology, 226, 221-230

Carre, B.; Gomez, J. and Chagneau, A.M. (1995) Contribution of oligosaccharide and polysaccharide digestion, and excreta losses of lactic acid and short chain fatty acids, to dietary metabolisable energy value in broiler chickens and adult cockerels. Br. Poultry. Sci., 36:611-629.

Carvalheiro F., Esteves, M.P., Parajo J.C., Pereira, H., Girio, F.M. (2004) Production of oligosaccharides by autohydrolysis of brewery's spent grain. Bioresource Technol 91: 93-100

Choct M. and Kocher A. (2000) Non-starch carbohydrates: Digestion ans its secondaary effects in monogastrics. Proceedings of 24th Annual Meeting of the Nutrition Society of Australia, Fremantle, Perth, pp 31-38.

Choct, M. and Annison, G. (1992) Br. Poult. Sci. 33: 821.

Cleemput, G., Roels, S.P., Van Oort, M., Grobet, P.J. and Delcour, J.A. (1993). Heterogeneity in the structure of water-soluble arabinoxylans in European wheat flours of variable bread-making quality. Cereal Chem., 70(3): 324-329.

Coon C.N., Leske K.L., Akavanichan O. and Cheng T.K. (1990) Effect of oligosaccharide-free soybean meal on true metabolizable energy and fiber digestion in adult roosters. Poult Sci , 69, 787-793.

Courtin, C.M. and Delcour, J.A. (1998). Physicochemical and bread-making properties of low molecular weight wheat-derived arabinoxylans. J. Agric. Food Chem., 46: 4066-4073.

Courtin, C.M. and Delcour, J.A.,(1998) Physico-chemical and breadmaking properties of low molecular weight wheat derived arabinoxylans, J. Agric. Food Chem., 46, 4066 - 4073.

Courtin, C.M., Van den Broeck, H. and Delcour, J.A. (2000). Determination of reducing end sugar residues in oligo- and polysaccharides by gas liquid chromatography. Journal of Chromatography A, 866, 97-104.

De Preter V, Geboes K, Verbrugghe K, De Vuyst L, Vanhoutte T, Huys G, Swings J, Pot B, Verbeke K (2004) The in vivo use of the stable isotope-labelled biomarkers lactose-[15N]ureide and [2H4]tyrosine to assess the effects of pro- and prebiotics on the intestinal flora of healthy human volunteers. Brit J Nutrition 92: 439-446.

Evenepoel P, Claus D, Geypens B et al. (1999) Amount and fate of egg protein escaping assimilation in the small intestine of humans. Am J Physiol 277, G935-G943.

Fincher, G.B., Stone, B.A., Clarke, A.E.(1983) Arabinogalactan-proteins: structure, biosynthesis, and function, Ann. Rev. Plant Physiol., 34, 47-70. Fooks LJ, Fuller R & Gibson GR (1999) Prebiotics, probiotics and human gut microbiology. Int Dairy J 9. 53-61

Gdala , J.; Jansman .J.M.; Buraczewska, L.; Huisman, J. and van Leeuwen, P. (1997) The influence of alpha-galactosidase supplementation on the ileal digestibility of lupin seed carbohydrates and dietary protein in young pigs. Anim. Feed Sci. Tech., 67:115-125.

Geboes K.P., De Peter V, Luypaerts A., Bammens B., Evenepoel P., Ghoos Y., Rutgeerts P., Verbeke K. (2005) Validation of lactose[15N,15N]ureide as a tool to study colonic nitrogen metabolism. Am J Physiol 288: G994-G999.

Gibson , G.R. and Roberfroid M.B. (1995) Dietary modulation of the human colonic microbiota: introducing the concept of prebiotics. J. Nutr. 125: 1401-1412.

Grasten S, Liukkonen K-H, Chrevatidis A, El-Nezami H, Poutanen K, Mykkanen H (2003) Effects of wheat pentosan and inulin on the metabolic activity of fecal microbiota and on bowel function in healthy humans. Nutrition Research 23: 1503-1514.

Hofmann E (1931) Ueber den Abbau von glucoseureid durch Bakterien. Biochem Zeitschr 243, 416-422

Hoseny, R.C. (1994) Principals of cereal science and technology. AACC, St. Paul, MN, USA.

Hsu C-K, Liao, J-W, Chung, Y-C, Hsieh, C-P, Chan, Y-C (2004) Xylooligosaccharides and fructooligosaccharides affect the intestinal microbiota and precancerous colonic lesions development in rats. J. Nutr. 134:1523-1528

Iji, P.A. (1999) The impact of cereal non-starch polysaccharides on intestinal development and function in broiler chickens. Wld.'s Poult. Sci. J., 55:375-387.

Kabel, MA., Kortenoeven, L., Schols, H.A. & Voragen, A.G.J. (2002). In vitro fermentability of differently substituted xylo-oligosaccharides. Journal of Agricultural and Food Chemistry, 50: 6205-6210.

Kocher, A. Hughes, R.J. and Choct, M. (1999) Lupin oligosaccharides: nutrients and anti-nutrients? Proc. Of Australian Poultr. Sci. Symp.; Sydney, Australia,.

Maes, C., Vangeneugden, B. and Delcour, J.A. (2004). Relative activity of two endoxylanases towards water-unextractable arabinoxylans in wheat bran. J. Cereal Sci., 39: 181-186.

McRitchie, F. (1985). Studies of the methodology for fractionation and reconstitution of wheat flour. J. Cereal Sci., 3: 221-230.

Morris, E.R. (1984) Rheology of hydrocolloids in gums and stabilisers for the food industry. Phillips, G.O., Wedlock, D.J., Williams P.A., Eds.; Pergamon Press: Oxford, U.K.

Naveed, A. (1999) The effect of enzyme supplementation of UK grown lupin seeds on growth and nutrient digestibility in broilers. MSc. Thesis , University of Aberdeen.

Okazaki M., Fujikawa, S., Matsumoto, N. (1990) Effect of xylooligosaccharides on the growth of bifidobacteria. Bifidobacteria Microflora 9:77-86

Rada, V. and Petr, J. (2000) A new selective medium for the isolation of glucose non-fermenting bifidobacteria from hen caeca. Journal of Microbiological Methods 43, 127-132.

Rada, V., Sirotek, K. and Petr, J. (1999) Evaluation of selective media for bifodobacteria in poultry and rabbit caecal samples. Journal of Veterinary Medicine B 46, 369-373,

Roberfroid, M.B. (1998) Prebiotics and synbiotics: concepts and nutritional properties. Brit. J. Nutr. 80:S197-S202. Saini, H.S. (1989) Legum seed oligosaccharides Prc Rec Adv Res Antinutritive Fact Legume Seed, Wageningen, 329-341.

Santori J., Potthast A., Ecker A., Sixta H., Rosenau T., Kosma P. (2003). Alkaline degradation kinetics and CE-separation of cello- and xylo-oligomers. Carbohydrate Research, 338 : 1209-1216.

Satokari, R.M., Vaughan, E.E., Akkermans, A.D.L., Saarela, M. and De Vos, W.M. (2001) Bifidobacterial diversity in human feces detected by genus-specific PCR and denaturing gradient gel electrophoresis. Appl. Env. Micorbiol. 67:504-513.

Savory, C.J. (1992b) Enzyme supplementation, degradation and metabolism of three U-14C-labelled cell-wall substrates in the fowl. Br. J. Nutr, 67:91-102.

Savory, C.J. (1992a) Gastro-intestinal morphology and absorption of monosaccharides in fowls conditioned to different types and levels of dietary fibre.

Smith EA and Macfarlane GT (1996) Enumeration of human colonic bacteria producing phenolic and indolic compounds: effects of pH, carbohydrate availability and retetion time on dissimilatory aromatic amino acid metabolism.

J Applied Bacteriol. 81: 288-302

Snedecor, G.W., and W.G. Cochran, (1989). Statistical methods (8th edn.). Iowa State University Press. Ames, IA, USA.

Spring P., Wenk C., Dawson K.A. and Newman K.E. (2001) The effects of dietary mannaoligosaccharides on cecal parameters and the concentrations of enteric bacteria in the ceca of salmonella-challenged broiler chicks. Poult Sci, 79, 205 - 211.

Statgraphics version 6.1, (1992). Reference Manual. Statistical Graphics Corporation (Rockville, M.D., USA). Strahm, A., Amado, R., Neukom, H., (1981) Hydroxyproline-galactoside as a protein-polysaccharide linkage in a water soluble arabinogalactan-peptide from wheat endosperm, Phytochem. 20, 1061-1063.

Suntory. Xylo-oligosaccharide, brochure and product sheet, 2001

Teitelbaum, J.E., Walker, W.A. (2002) Nutritional impact of pre- and probiotics as protective gastrointestinal organisms. Annual Review of Nutrition 22: 107-38

Topping DL and Clifton PM (2001) Short-chain fatty acids and human colonic function: roles of resistant starch and nonstarch polysaccharides. Physiol Rev 81:1031-1064

Toyoda, Y., Hatakana, Y., Suwa, Y., (1993) Effect of xylooligosaccharides on calcium absorption. In Proc. of 47th Annual Meeting of Jpn Soc Nutr Food Sci, Tokyo, p109

Vahouny, G.V., (1982) Fed.Proc. 41:2801.

Van de Wiele, T., Boon, N., Possemiers, S., Jacobs, H., Verstraete, W (2004). Prebiotic effects of chicory inulin in the simulator of the human intestinal microbial ecosystem. FEMS Microbiol. Ecol. 51:143-153.

Van Loo, J.A.E. (2004) Prebiotics promote good health. The basis, the potential, and the emerging evidence. J Clin Gastroenterol 38: S70-S75.

Vinkx, C.J.A., Van Nieuwenhove, C.G., Delcour, J.A. (1991). Physico-chemical and functional properties of rye nonstarch polysaccharides. III. Oxidative gelation of a fraction containing water soluble pentosans and proteins. Cereal Chem. 68: 617-622.

Visek WJ. (1978) Diet and cell growth modulation by ammonia. Am J Clin Nutr 31, Supp 10: S216-S220.

Whistler, R.L., BeMiller, J.N. (1958) Alkaline degradation of polysaccharides. Adv. Carbohydr. Chem., 13, 289-389

Wutzke KD, Heine WE, Plath C, Leitzmann P, Radke M, Mohr C, Richter I, Gulzow HU, and Hobusch D. (1997) Evaluation of OCTT: a comparison of the lactose-[13C,15N]-ureide, 13CO2- and the lactulose H2-breath test in humans. Eur J Clin Nutr 51: 11-19.

Yamada H., Itoh, K., Morishita, Y., Taniguchi, H. (1993) Structure and properties of oligosaccharides from wheat bran. Cereal Foods World 38: 490-492

Zdunczyk, Z.; Juskiewicz, J., Frejnagel S. and Gulewicz, K. (1998) Influence of alkaloids and oligosaccha-rides from white lupin seeds utilisation of diets by rats and absorption of nutrients in the small intestine. Anim. Feed Sci. Tech. 72:143-154.

Zyla, K., Gogal, D.; Koreleski, J.; Swiatkiewicz, S and Ledoux, D.R. (1999b) Simultaneous application of phytase and xylanase to broiler feeds based on wheat: feeding experiment with growing broilers. J. Sci. Food Agric. 79: 1841-1848

Zyla , K., Gogal, D.; Koreleski, J.; Swiatkiewicz, S. and Ledoux, D.R. (1999a) Simultaneous application of phyatse and xylanase to broiler feeds based on wheat: in vitro measurements of phosphorus and pentose release from wheat and wheat-based feeds. J. Sci. Food Agric., 79:1832-1840.

**TABLES**

Table 1: Properties of different (arabino)xylan preparations. AX: arabinoxylan content expressed as % of dry matter; A/X: the arabinose to xylose ratio; $avDP_{GC}$: average degree of polymerisation as determined by gas-liquid chromatography; $avDP_{HPSEC}$: average degree of polymerisation as determined by high performance size exclusion chromatography. DP 90% range: range of degrees of polymerisation within which 90% of the oligosaccharides fall.

| | AX[a] | A/X | $avDP_{GC}$ | $aVDP_{HPSEC}$[b] | DP 90% range |
|---|---|---|---|---|---|
| WPC | 42.8[d] | 0.58[e] | N.D. | 58 | 1-480 |
| AXOS-122-0.66 | 73.7[d] | 0.66[e] | 122 | 150 | 12-1200 |
| AXOS-7-0.34 | 67.8 | 0.34 | 7 | 9 | 2-190 |
| AXOS-16-0.78 | 77.6 | 0.78 | 16 | 19 | 2-130 |
| AXOS-15-0.27 | 72.3 | 0.27 | 15 | 12 | 2-650 |
| AXOS-8-0.27 | 80.4 | 0.27 | 8 | N.D. | N.D. |
| AXOS-39-0.22 | 77.3 | 0.22 | 39 | 46 | 5-240 |
| AXOS-13-0.21 | 86.2 | 0.21 | 13 | 9 | 2-80 |
| Xylooligo-95P | 79.2 | 0.09 | 2 | 3 | 1-5 |

[a] AX=0.88 x (% arabinose + % xylose)
[b] $avDP_{HPSEC} = MM_{HPSEC}/132$
[c] N.D. = Not determined
[d] $AX_{cor} = 0.88$ x (% arabinose - 0.7 x % galactose + % xylose)
[e] $A/X_{cor} = $ (% arabinose - 0.7 x % galactose) /% xylose

Table 2: Concentrations of different compounds used for sensory analysis tests.

| Concentration number | AXOS-15-0.27 (g/L) | Xylooligo-95P (g/L) | Sucrose (g/L) | Sodium chloride ($\mu$g/L) | Ascorbic acid ($\mu$g/L) |
|---|---|---|---|---|---|
| 8 | 71.5 | 64 | 32.4 | 800 | 96 |
| 7 | 35.7 | 32 | 16.2 | 400 | 48 |
| 6 | 17.9 | 16 | 8.1 | 200 | 24 |
| 5 | 8.9 | 8 | 4.0 | 100 | 12 |
| 4 | 4.5 | 4 | 2.0 | 50 | 6 |
| 3 | 2.2 | 2 | 1.0 | 25 | 3 |
| 2 | 1.1 | 1 | 0.5 | 12.5 | 1.5 |
| 1 | 0.5 | 0.5 | 0.25 | 6.25 | 0.75 |

Table 3. Apparent intrinsic viscosity (dl/g) of a 5% (w/v) solution of different oligosaccharides.

| | Apparent intrinsic viscosity (dl/g) |
|---|---|
| FOS (Raftilose) | 0.037 |
| Xylooligo-95P | 0.045 |
| AXOS-15-0.27 | 0.515 |
| AXOS-39-0.22 | 0.281 |
| AXOS-13-0.21 | 0.130 |

Table 4: Ultrafiltration of AXOS preparation by passing through a single membrane with a MMCO of either 5 kDa, 10 kDa, or 30 kDa. Recovery is expressed as % of the arabinoxylan content of the starting material. The analyses include the arabinose to xylose ratio (A/X), and the average degree of polymerisation as determined by gas-liquid chromatography ($avDP_{GC}$).

| Fraction | Recovery | A/X | $avDP_{GC}$ |
|---|---|---|---|
| AXOS before ultrafiltration | 100% | 0.50 | 8 |
| AXOS in permeate of 5 kDa membrane | 11.2% | 0.24 | 3 |
| AXOS in retentate of 5 kDa membrane | 86.1% | 0.55 | 11 |
| AXOS in permeate of 10 kDa membrane | 34.2% | 0.33 | 5 |
| AXOS in retentate of 10 kDa membrane | 64.8% | 0.58 | 12 |
| AXOS in permeate of 30 kDa membrane | 52.4% | 0.40 | 6 |
| AXOS in retentate of 30 kDa membrane | 45.7% | 0.61 | 15 |

Table 5: Ultrafiltration of AXOS preparation by passing consecutively through membranes of 10 and 30 kDa. Recovery is expressed as % of the arabinoxylan content of the starting material. The analyses include the arabinose to xylose ratio (A/X), and the average degree of polymerisation as determined by gas-liquid chromatography ($avDP_{GC}$).

| Fraction | Recovery | A/X | $avDP_{GC}$ |
|---|---|---|---|
| AXOS before ultrafiltration | 100% | 0.50 | 8 |
| AXOS after passing through 10kDa membrane (permeate) | 34.2% | 0.33 | 5 |
| AXOS after passing consecutively through10kDa membrane (retentate) and 30kDa membrane (permeate) | 25.2% | 0.50 | 7 |
| AXOS after passing consecutively through10kDa membrane (retentate) and 30kDa membrane (retentate) | 36.6% | 0.65 | 18 |

Table 6: Feedstuffs and compositional analysis of the chicken feed diet used in the second chicken experiment.

| feedstuffs | % |
|---|---|
| Wheat | 40.72 |
| yellow corn | 17.00 |
| soybean meal-48 | 21.81 |
| HF soybeans | 10.00 |
| RA fat | 6.54 |
| CaCO3 | 0.85 |

(continued)

| feedstuffs | % |
|---|---|
| D-Ca-phosphate | 1.08 |
| NaCl | 0.32 |
| R phytase | 0.035 |
| L lys HCl | 0.29 |
| DL methionine | 0.26 |
| L threonine | 0.10 |
| Vit. & Tr. el. premix | 1.00 |
| **Sum** | 100.005 |
| **composition** | |
| Metabolisable Energy, MJ/kg | 12.30 |
| Crude protein, % | 21.00 |
| d-lys, % | 1.15 |
| d-SAA, % | 0.80 |
| d-thr, % | 0.75 |
| Ca, % | 0.95 |
| Pav., % | 0.45 |
| Na+K-Cl, meq/kg | 214 |
| C18:2, % | 2.29 |

Table 7: Compositional analysis of the 'basic humanised diet' used in the rat experiments.

| Composition | |
|---|---|
| Metabolisable Energy (MJ/kg) | 18.7 |
| Dry matter, % | 94.4 |
| Crude protein, % | 20.4 |
| Crude fat, % | 19.2 |
| Crude fibre, % | 1.4 |
| Crude ash, % | 6.2 |
| N free extracts, % | 47.3 |
| Starch, % | 28.2 |
| Sucrose, % | 16.7 |
| Calcium, % | 0.94 |
| Phosphorus, % | 0.70 |
| Sodium, % | 0.35 |
| Magnesium, % | 0.18 |
| Potassium, % | 0.31 |
| Chloride, % | 0.5 |

## Claims

1. A food or beverage product modulating the human intestinal flora comprising between 0.25 and 5 g of arabinoxylans per serving of said food or beverage product wherein said arabinoxylans have an average degree of polymerisation (DP) between 5 and 50.

2. A food or beverage product according to claim 1, wherein said arabinoxylans have an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50.

3. The food or beverage product according to claim 1 or claim 2, comprising between 1 and 3 g of said arabinoxylans per serving of said food or beverage product.

4. The food product according to any of claims 1 to 3, wherein the food product is a meat product or a dairy product.

5. The beverage according to any of claims 1 to 3, wherein the beverage is a non-alcoholic beverage or a functional soft drink.

6. The beverage or food product according to any of claims 1 to 5, wherein said food product comprises living bacteria of the genus Bifidobacterium or Lactobacillus.

7. The food product according to claim 1, wherein said food product is a cereal-containing food product comprising between 0.25 and 5 g of arabinoxylans having a DP below 200 per serving of said product and wherein said population of arabinoxylans has an average DP between 5 and 50.

8. The food product according to claim 2, wherein said food product is a cereal-containing food product comprising between 0.25 and 5 g of arabinoxylans having a DP below 200 per serving of said product and wherein said population of arabinoxylans has an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50.

9. The cereal-containing food product according to claim 7 or claim 8, comprising between 1 and 3 g of said arabinoxylans.

10. The food product according to any of claims 7 to 9, wherein the food product is a bakery or pastry product.

11. The food product according to any of claims 7 to 9, wherein the food product is a breakfast cereal, a biscuit or a pasta product.

12. The food product according to any of claims 1 to 6, wherein the food product is an industrially prepared dessert product.

13. The food or beverage product according to any claims 1 to 6, wherein the food or beverage product is a low calorie product.

14. A method for the preparation of a nutritional additive to be used as an additive in the preparation of a food product according to any of claims 1 to 13, comprising the steps of:

   i. isolating the water unextractable arabinoxylan fraction from an arabinoxylan-rich material such as bran, seed flour or seed squeegee by destarchification and deproteinisation of said arabinoxylan rich material preferably using amylolytic and proteolytic enzymes, respectively, wherein the seed is wheat, barley, rye, oats, sorghum, and rice and
   ii. depolymerising the water unextractable arabinoxylan fraction using one or more xylanolitic enzymes.

15. A method for the preparation of a nutritional additive to be used as an additive in the preparation of a food product according to any of claims 1 to 13, comprising the steps of:

   i. obtaining a partially depolymerised water extractable arabinoxylan-containing preparation, such as a preparation obtained out of a side stream of the starch-gluten separation process

ii. if necessary eliminating the starch and proteins from said arabinoxylan-containing preparation, preferably using amylolytic and proteolytic enzymes, respectively;

iii. depolymerising the arabinoxylans contained in said preparation using one or more xylanolitic enzymes

16. The method according to claim 14 or claim 15, wherein the preparation is subjected to ultrafiltration, after the treatment with the xylanolytic enzymes in order to remove low molecular weight compounds having a DP of 4 or less.

17. A nutritional additive to be used as an additive in the preparation of a food product or beverage according to claim 1, wherein said additive comprises at least 15% arabinoxylans having an average degree of polymerisation (DP) between 7 and 20.

18. A nutritional additive to be used as an additive in the preparation of a food product or beverage according to any of claims 1 to 13, wherein said additive comprises at least 15% arabinoxylans, wherein said arabinoxylans have an average degree of polymerisation (DP) of 5 or 7, or said arabinoxylans have an average degree of polymerisation (DP) of 6 when their A/X (arabinose to xylose) ratio is 0.40, or said arabinoxylans have an average degree of polymerisation (DP) of 8 when their A/X (arabinose to xylose) ratio is 0.50.

19. The use of an arabinoxylan preparation comprising arabinoxylan molecules with an average degree of polymerisation (DP) varying between 5 and 50 as a food additive in the production of a food or beverage, which comprises between 0.25 and 5 g of such arabinoxylans per serving of said food or beverage

20. The use of an arabinoxylan preparation according to claim 19, wherein the arabinoxylan molecules have an average degree of polymerisation (DP) of 5 or 7, or an average degree of polymerisation (DP) of 6 when their A/X (arabinose to xylose) ratio is 0.40, or an average degree of polymerisation (DP) of 8 when their A/X (arabinose to xylose) ratio is 0.50, as a food additive in the production of a food or beverage, which comprises between 0.25 and 5 g of such arabinoxylans per serving of said food or beverage.

21. The use of an arabinoxylan preparation according, to claim 19 or claim 20 in the preparation of a food or beverage comprising between 1 and 3 g of said arabinoxylans per serving of said food or beverage.

22. The use of an arabinoxylan preparation according to any of claims 19 to 21, wherein the food is a meat product or a dairy product.

23. The use of an arabinoxylan preparation according to any of claims 19 to 21, wherein the beverage is a non-alcoholic beverage.

24. The use of an arabinoxylan preparation according to any of claims 19 to 21, wherein the food product is a bakery or a pastry product.

25. The use of an arabinoxylan preparation according to any of claims 19 to 21, wherein the food product is a pasta product, a biscuit or a breakfast cereal.

26. The use of an arabinoxylan preparation according to claim 22 or claim 23, wherein the food or beverage product comprises a living bacterium of the genus Bifidobacterium or Lactobacillus.

**Patentansprüche**

1. Nahrungs- oder Getränkeprodukt, das die menschliche Darmflora moduliert, umfassend zwischen 0,25 und 5 g Arabinoxylane pro Portion des Nahrungs- oder Getränkeprodukts, wobei die Arabinoxylane einen durchschnittlichen Polymerisationsgrad (DP) zwischen 5 und 50 aufweisen.

2. Nahrungs- oder Getränkeprodukt nach Anspruch 1, wobei die Arabinoxylane einen durchschnittlichen Polymerisationsgrad (DP) von 5 oder 7 aufweisen, oder die Arabinoxylane einen durchschnittlichen Polymerisationsgrad (DP) von 6 aufweisen, wenn ihr A/X(Arabinose zu Xylose)-Verhältnis 0,40 beträgt, oder die Arabinoxylane einen durchschnittlichen Polymerisationsgrad (DP) von 8 aufweisen, wenn ihr A/X(Arabinose zu Xylose)-Verhältnis 0,50 beträgt.

3. Nahrungs- oder Getränkeprodukt nach Anspruch 1 oder 2, das zwischen 1 und 3 g der Arabinoxylane pro Portion des Nahrungs- oder Getränkeprodukts umfasst.

4. Nahrungsprodukt nach einem der Ansprüche 1 bis 3, wobei das Nahrungsprodukt ein Fleisch- oder Milchprodukt ist.

5. Getränk nach einem der Ansprüche 1 bis 3, wobei das Getränk ein nicht-alkoholisches Getränk oder ein funktioneller Softdrink ist.

6. Getränke- oder Nahrungsprodukt nach einem der Ansprüche 1 bis 5, wobei das Nahrungsprodukt lebende Bakterien der Gattung *Bifidobacterium* oder *Lactobacillus* umfasst.

7. Nahrungsprodukt nach Anspruch 1, wobei das Nahrungsprodukt ein Cerealien-enthaltendes Nahrungsprodukt ist, das zwischen 0,25 und 5 g Arabinoxylane mit einem DP unter 200 pro Portion des Produkts umfasst, wobei die Population von Arabinoxylanen einen durchschnittlichen DP zwischen 5 und 50 aufweist.

8. Nahrungsprodukt nach Anspruch 2, wobei das Nahrungsprodukt ein Cerealien-enthaltendes Nahrungsprodukt ist, das zwischen 0,25 und 5 g Arabinoxylane mit einem DP unter 200 pro Portion des Produkts umfasst und wobei die Population von Arabinoxylanen einen durchschnittlichen Polymerisationsgrad (DP) von 5 oder 7 aufweist, oder die Arabinoxylane einen durchschnittlichen Polymerisationsgrad (DP) von 6 aufweisen, wenn ihr A/X(Arabinose zu Xylose)-Verhältnis 0,40 beträgt, oder die Arabinoxylane einen durchschnittlichen Polymerisationsgrad (DP) von 8 aufweisen, wenn ihr A/X(Arabinose zu Xylose)-Verhältnis 0,50 beträgt.

9. Cerealien-enthaltendes Nahrungsprodukt nach Anspruch 7 oder 8, das zwischen 1 und 3 g der Arabinoxylane umfasst.

10. Nahrungsprodukt nach einem der Ansprüche 7 bis 9, wobei das Nahrungsprodukt ein Bäckerei- oder Konditoreiprodukt ist.

11. Nahrungsprodukt nach einem der Ansprüche 7 bis 9, wobei das Nahrungsprodukt eine Frühstückscerealie, ein Keks- oder ein Pastaprodukt ist.

12. Nahrungsprodukt nach einem der Ansprüche 1 bis 6, wobei das Nahrungsprodukt ein industriell hergestelltes Dessertprodukt ist.

13. Nahrungs- oder Getränkeprodukt nach einem der Ansprüche 1 bis 6, wobei das Nahrungs- oder Getränkeprodukt ein kalorienarmes Produkt ist.

14. Verfahren zur Herstellung eines Nahrungsadditivs, das als Additiv bei der Herstellung eines Nahrungsprodukts nach einem der Ansprüche 1 bis 13 zu verwenden ist, umfassend die Schritte:

   i. Isolieren der wasser-unextrahierbaren Arabinoxylan-Fraktion aus einem arabinoxylanreichen Material, wie Kleie, Samenmehl oder Samen-Quetschmasse, durch Entstärkung und Deproteinisierung des arabinoxylanreichen Materials, vorzugsweise unter Verwendung von amylolytischen bzw. proteolytischen Enzymen, wobei der Samen Weizen, Gerste, Roggen, Hafer, Hirse und Reis ist, und
   ii. Depolymerisieren der wasser-unextrahierbaren Arabinoxylan-Fraktion unter Verwendung von einem oder mehreren xylanolytischen Enzymen.

15. Verfahren zur Herstellung eines Nahrungsadditivs, das als Additiv bei der Herstellung eines Nahrungsprodukts nach einem der Ansprüche 1 bis 13 zu verwenden ist, umfassend die Schritte:

   i. Erhalten einer teilweise depolymerisierten wasser-extrahierbaren Arabinoxylanenthaltenden Präparation, wie eine Präparation, die aus einem Nebenstrom des Stärke-Gluten-Trennverfahrens erhalten wird;
   ii. sofern notwendig, Beseitigen der Stärke und Proteine aus der Arabinoxylanenthaltenden Präparation, vorzugsweise unter Verwendung von amylolytischen bzw. proteolytischen Enzymen;
   iii. Depolymerisieren der in der Präparation enthaltenen Arabinoxylane unter Verwendung von einem oder mehreren xylanolytischen Enzymen.

16. Verfahren nach einem der Ansprüche 14 oder 15, wobei die Präparation einer Ultrafiltration nach der Behandlung mit den xylanolytischen Enzymen unterzogen wird, um niedermolekulare Verbindungen mit einem DP von 4 oder weniger zu entfernen.

17. Nahrungsadditiv, das als Additiv bei der Herstellung eines Nahrungsprodukts oder Getränks nach Anspruch 1 zu verwenden ist, wobei das Additiv mindestens 15 % Arabinoxylane mit einem durchschnittlichen Polymerisationsgrad (DP) zwischen 7 und 20 umfasst.

18. Nahrungsadditiv, das als Additiv bei der Herstellung eines Nahrungsprodukts oder Getränks nach einem der Ansprüche 1 bis 13 zu verwenden ist, wobei das Additiv mindestens 15 % Arabinoxylane umfasst, wobei die Arabinoxylane einen durchschnittlichen Polymerisationsgrad (DP) von 5 bis 7 aufweisen, oder die Arabinoxylane einen durchschnittlichen Polymerisationsgrad (DP) von 6 aufweisen, wenn ihr A/X(Arabinose zu Xylose)-Verhältnis 0,40 beträgt, oder die Arabinoxylane einen durchschnittlichen Polymerisationsgrad (DP) von 8 aufweisen, wenn ihr A/X (Arabinose zu Xylose)-Verhältnis 0,50 beträgt.

19. Verwendung einer Arabinoxylan-Präparation, die Arabinoxylan-Moleküle mit einem durchschnittlichen Polymerisationsgrad (DP) umfasst, der zwischen 5 und 50 Grad schwankt, als ein Nahrungsadditiv bei der Herstellung eines Nahrungsmittels oder Getränks, das zwischen 0,25 und 5 g solcher Arabinoxylane pro Portion des Nahrungsmittels oder Getränks umfasst.

20. Verwendung einer Arabinoxylan-Präparation nach Anspruch 19, wobei die Arabinoxylan-Moleküle einen durchschnittlichen Polymerisationsgrad (DP) von 5 oder 7 aufweisen, oder einen durchschnittlichen Polymerisationsgrad (DP) von 6 aufweisen, wenn ihr A/X(Arabinose zu Xylose)-Verhältnis 0,40 beträgt, oder einen durchschnittlichen Polymerisationsgrad (DP) von 8 aufweisen, wenn ihr A/X(Arabinose zu Xylose)-Verhältnis 0,50 beträgt, als Nahrungsadditiv bei der Herstellung eines Nahrungsmittels oder Getränks, das zwischen 0,25 und 5 g solcher Arabinoxylane pro Portion des Nahrungsmittels oder Getränks umfasst.

21. Verwendung einer Arabinoxylan-Präparation nach Anspruch 19 oder Anspruch 20 bei der Herstellung eines Nahrungsmittels oder Getränks, das zwischen 1 und 3 g der Arabinoxylane pro Portion des Nahrungsmittels oder Getränks umfasst.

22. Verwendung einer Arabinoxylan-Präparation nach einem der Ansprüche 19 bis 21, wobei das Nahrungsmittel ein Fleisch oder Milchprodukt ist.

23. Verwendung einer Arabinoxylan-Präparation nach einem der Ansprüche 19 bis 21, wobei das Getränk ein nicht-alkoholisches Getränk ist.

24. Verwendung einer Arabinoxylan-Präparation nach einem der Ansprüche 19 bis 21, wobei das Nahrungsprodukt ein Bäckerei- oder Konditoreiprodukt ist.

25. Verwendung einer Arabinoxylan-Präparation nach einem der Ansprüche 19 bis 21, wobei das Nahrungsprodukt ein Pastaprodukt, ein Keks oder eine Frühstückscerealie ist.

26. Verwendung einer Arabinoxylan-Präparation nach Anspruch 22 oder 23, wobei das Nahrungs- oder Getränkeprodukt ein lebendes Bakterium der Gattung *Bifidobacterium* oder *Lactobacillus* umfasst.

**Revendications**

1. Produit alimentaire ou boisson modulant la flore intestinale humaine, comprenant entre 0,25 et 5 g d'arabinoxylanes par portion dudit produit alimentaire dans lequel lesdits arabinoxylanes ont un degré moyen de polymérisation (DP) compris entre 5 et 50.

2. Produit alimentaire ou boisson selon la revendication 1, dans lequel lesdits arabinoxylanes ont un degré moyen de polymérisation (DP) de 5 ou de 7, ou lesdits arabinoxylanes ont un degré moyen de polymérisation (DP) de 6 lorsque leur rapport A/X (arabinose au xylose) est de 0,40, ou bien lesdits arabinoxylanes ont un degré moyen de polymérisation (DP) de 8 lorsque leur rapport A/X (arabinose au xylose) est de 0,50.

3. Produit alimentaire ou boisson selon la revendication 1 ou la revendication 2, comprenant entre 1 et 3 g desdits arabinoxylanes par portion dudit produit alimentaire ou de ladite boisson.

4. Produit alimentaire selon l'une quelconque des revendications 1 à 3, ledit produit alimentaire étant un produit à base de viande ou un produit laitier.

5. Boisson selon l'une quelconque des revendications 1 à 3, ladite boisson étant une boisson sans alcool ou une boisson non alcoolisée fonctionnelle.

6. Boisson ou produit alimentaire selon l'une quelconque des revendications 1 à 5, ledit produit alimentaire comprenant des bactéries vivantes du genre Bifidobacterium ou Lactobacillus.

7. Produit alimentaire selon la revendication 1, ledit produit alimentaire étant un produit alimentaire contenant des céréales comprenant entre 0,25 et 5 g d'arabinoxylanes ayant un DP au-dessous de 200 par portion dudit produit et dans lequel ladite population d'arabinoxylanes a un DP moyen compris entre 5 et 50.

8. Produit alimentaire selon la revendication 2, ledit produit alimentaire étant un produit alimentaire contenant des céréales comprenant entre 0,25 et 5 g d'arabinoxylanes ayant un DP au-dessous de 200 par portion dudit produit et dans lequel ladite population d'arabinoxylanes a un degré moyen de polymérisation (DP) de 5 ou de 7, ou lesdits arabinoxylanes ont un degré moyen de polymérisation (DP) de 6 lorsque leur rapport A/X (arabinose au xylose) est de 0,40, ou bien lesdits arabinoxylanes ont un degré moyen de polymérisation (DP) de 8 lorsque leur rapport A/X (arabinose au xylose) est de 0,50.

9. Produit alimentaire contenant des céréales selon la revendication 7 ou la revendication 8, comprenant entre 1 et 3 g desdits arabinoxylanes.

10. Produit alimentaire selon l'une quelconque des revendications 7 à 9, ledit produit alimentaire étant un produit de boulangerie ou de pâtisserie.

11. Produit alimentaire selon l'une quelconque des revendications 7 à 9, ledit produit alimentaire étant une céréale pour le petit déjeuner, un biscuit ou un produit à base de pâtes alimentaires.

12. Produit alimentaire selon l'une quelconque des revendications 1 à 6, ledit produit alimentaire étant un dessert préparé industriellement.

13. Produit alimentaire ou boisson selon l'une quelconque des revendications 1 à 6, ledit produit alimentaire ou ladite boisson étant un produit hypocalorique.

14. Procédé pour la préparation d'un additif nutritionnel devant être utilisé en tant qu'additif dans la préparation d'un produit alimentaire selon l'une quelconque des revendications 1 à 13, comprenant les étapes consistant à :

    i. isoler la fraction d'arabinoxylanes non extractible à l'eau d'un matériau riche en arabinoxylanes tel que du son, de la farine de grain ou du grain de 'squeegee' en désamidonnant et en déprotéinisant ledit matériau riche en arabinoxylanes, de préférence en utilisant, respectivement, des enzymes amylolytiques et protéolytiques lorsque le grain est du blé, de l'orge, du seigle, de l'avoine, du sorgho et du riz, et
    ii. dépolymériser la fraction d'arabinoxylanes non extractible à l'eau en utilisant une ou plusieurs enzymes xylanolytiques.

15. Procédé pour la préparation d'un additif nutritionnel devant être utilisé en tant qu'additif dans la préparation d'un produit alimentaire selon l'une quelconque des revendications 1 à 13, comprenant les étapes consistant à:

    i. obtenir une préparation contenant des arabinoxylanes partiellement dépolymérisés extractibles à l'eau, une telle préparation étant obtenue à la sortie d'un courant latéral du procédé de séparation amidon-gluten ;
    ii. si nécessaire, éliminer l'amidon et les protéines de ladite préparation contenant des arabinoxylanes, de préférence en utilisant, respectivement, des enzymes amylolytiques et protéolytiques ;
    iii. dépolymériser les arabinoxylanes contenus dans ladite préparation en utilisant une ou plusieurs enzymes xylanolytiques.

16. Procédé selon la revendication 14 ou la revendication 15, dans lequel la préparation est soumise à une ultrafiltration, après le traitement avec les enzymes xylanolytiques, afin d'éliminer les composés de faible poids moléculaire ayant un DP de 4 ou inférieur.

17. Additif nutritionnel devant être utilisé en tant qu'additif dans la préparation d'un produit alimentaire ou d'une boisson selon la revendication 1, ledit additif comprenant au moins 15 % d'arabinoxylanes ayant un degré moyen de polymérisation (DP) compris entre 7 et 20.

18. Additif nutritionnel devant être utilisé en tant qu'additif dans la préparation d'un produit alimentaire ou d'une boisson selon l'une quelconque des revendications 1 à 13, ledit additif comprenant au moins 15 % d'arabinoxylanes, dans lequel lesdits arabinoxylanes ont un degré moyen de polymérisation (DP) de 5 ou de 7, ou lesdits arabinoxylanes ont un degré moyen de polymérisation (DP) de 6 lorsque leur rapport A/X (arabinose au xylose) est de 0,40, ou bien lesdits arabinoxylanes ont un degré moyen de polymérisation (DP) de 8 lorsque leur rapport A/X (arabinose au xylose) est de 0,50.

19. Utilisation d'une préparation d'arabinoxylanes comprenant des molécules d'arabinoxylanes ayant un degré moyen de polymérisation (DP) variant entre 5 et 50 en tant qu'additif alimentaire dans la production d'un aliment ou d'une boisson, qui comprend entre 0,25 et 5 g de tels arabinoxylanes par portion dudit aliment ou de ladite boisson.

20. Utilisation d'une préparation d'arabinoxylanes selon la revendication 19, dans laquelle les molécules d'arabinoxylanes ont un degré moyen de polymérisation (DP) de 5 ou de 7, ou un degré moyen de polymérisation (DP) de 6 lorsque leur rapport A/X (arabinose au xylose) est de 0,40, ou bien un degré moyen de polymérisation (DP) de 8 lorsque leur rapport A/X (arabinose au xylose) est de 0,50, en tant qu'additif alimentaire dans la production d'un aliment ou d'une boisson, qui comprend entre 0,25 et 5 g de tels arabinoxylanes par portion dudit aliment ou de ladite boisson.

21. Utilisation d'une préparation d'arabinoxylanes selon la revendication 19 ou la revendication 20 dans la préparation d'un aliment ou d'une boisson comprenant entre 1 et 3 g desdits arabinoxylanes par portion dudit aliment ou de ladite boisson.

22. Utilisation d'une préparation d'arabinoxylanes selon l'une quelconque des revendications 19 à 21, dans laquelle l'aliment est un produit à base de viande ou un produit laitier.

23. Utilisation d'une préparation d'arabinoxylanes selon l'une quelconque des revendications 19 à 21, dans laquelle la boisson est une boisson non alcoolisée.

24. Utilisation d'une préparation d'arabinoxylanes selon l'une quelconque des revendications 19 à 21, dans laquelle le produit alimentaire est un produit de boulangerie ou de pâtisserie.

25. Utilisation d'une préparation d'arabinoxylanes selon l'une quelconque des revendications 19 à 21, dans laquelle le produit alimentaire est un produit à base de pâtes alimentaires, un biscuit ou une céréale pour le petit déjeuner.

26. Utilisation d'une préparation d'arabinoxylanes selon la revendication 22 ou la revendication 23, dans laquelle le produit alimentaire ou la boisson comprend une bactérie vivante du genre Bifidobacterium ou Lactobacillus.

Figures

Figure 1

Figure 2

Figure 3

Figure 4

Figure 5

Figure 6

Figure 7

Figure 8

Figure 9

Figure 10

Figure 11

A

B

C

D

Figure 12

Figure 13

41

Figure 14

A

B

C

Figure 15

A

B

Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0265970 B1
- EP 026597081 A
- US 2002037331 A

- US 5362502 A
- WO 02067698 A

### Non-patent literature cited in the description

- Official Methods of Analysis. Association of Official Analytical Chemists, 1995
- **BEAUGRAND J. ; CHAMBAT G. ; WONG V.W.K. ; GOUBET F. ; RÉMOND C. ; PAËS G. ; BENAMROUCHE S. ; DEBEIRE P. ; O'DONOHUE M. ; CHABBERT B.** Impact and efficiency of GH10 and GH11 thermostable endoxylanases on wheat bran and alkali-extractable arabinoxylans. *Carbohydr. Res.,* 2005, vol. 339, 2529-2540
- **BIELY, P. ; VRSANSKA, M. ; TENKANEN, M. ; KLUEPFEL, D.** J Biotechnol, 1997, vol. 57, 151-166
- **BONE E ; TAMM A ; HILL M.** The production of urinary phenols by gut bacteria and their possible role in the causation of large bowel cancer. *Am J Clin Nutr,* 1976, vol. 29, 1448-1454
- **BRITTON H.T.S ; WELFORD, G.** *J. Chem. Soc.,* 1937, 1848
- **CAMPBELL J.M. ; FAHEY, G.C. ; WOLF, B.W.** Selected indigestible oligosaccharides affect large bowel mass, cecal and fecal short-chain fatty acids, pH and micorflora in rats. *J. Nutr.,* 1997, vol. 127, 130-136
- **CAMPBELL, G.L. ; ROSSNAGEL, B.G. ; CLASSEN, H.L. ; THACKER, P.A.** Genotypic and environmental differences in extract viscosity of barley and their relationship to its nutritive value for broiler chickens. *Animal Feed Science and Technology,* 1989, vol. 226, 221-230
- **CARRE, B. ; GOMEZ, J. ; CHAGNEAU, A.M.** Contribution of oligosaccharide and polysaccharide digestion, and excreta losses of lactic acid and short chain fatty acids, to dietary metabolisable energy value in broiler chickens and adult cockerels. *Br. Poultry. Sci.,* 1995, vol. 36, 611-629
- **CARVALHEIRO F. ; ESTEVES, M.P. ; PARAJO J.C. ; PEREIRA, H. ; GIRIO, F.M.** Production of oligosaccharides by autohydrolysis of brewery's spent grain. *Bioresource Technol,* 2004, vol. 91, 93-100

- **CHOCT M. ; KOCHER A.** Non-starch carbohydrates: Digestion ans its secondaary effects in monogastrics. *Proceedings of 24th Annual Meeting of the Nutrition Society of Australia,* 2000, 31-38
- **CHOCT, M. ; ANNISON, G.** *Br. Poult. Sci.,* 1992, vol. 33, 821
- **CLEEMPUT, G. ; ROELS, S.P. ; VAN OORT, M. ; GROBET, P.J. ; DELCOUR, J.A.** Heterogeneity in the structure of water-soluble arabinoxylans in European wheat flours of variable bread-making quality. *Cereal Chem.,* 1993, vol. 70 (3), 324-329
- **COON C.N. ; LESKE K.L. ; AKAVANICHAN O. ; CHENG T.K.** Effect of oligosaccharide-free soybean meal on true metabolizable energy and fiber digestion in adult roosters. *Poult Sci,* 1990, vol. 69, 787-793
- **COURTIN, C.M. ; DELCOUR, J.A.** Physicochemical and bread-making properties of low molecular weight wheat-derived arabinoxylans. *J. Agric. Food Chem,* 1998, vol. 46, 4066-4073
- **COURTIN, C.M. ; DELCOUR, J.A.** Physico-chemical and breadmaking properties of low molecular weight wheat derived arabinoxylans. *J. Agric. Food Chem.,* 1998, vol. 46, 4066-4073
- **COURTIN, C.M. ; VAN DEN BROECK, H. ; DELCOUR, J.A.** Determination of reducing end sugar residues in oligo- and polysaccharides by gas liquid chromatography. *Journal of Chromatography A,* 2000, vol. 866, 97-104
- **DE PRETER V ; GEBOES K ; VERBRUGGHE K ; DE VUYST L ; VANHOUTTE T ; HUYS G ; SWINGS J ; POT B ; VERBEKE K.** The in vivo use of the stable isotope-labelled biomarkers lactose-[15N]ureide and [2H4]tyrosine to assess the effects of pro- and prebiotics on the intestinal flora of healthy human volunteers. *Brit J Nutrition,* 2004, vol. 92, 439-446
- **EVENEPOEL P ; CLAUS D ; GEYPENS B et al.** Amount and fate of egg protein escaping assimilation in the small intestine of humans. *Am J Physiol,* 1999, vol. 277, G935-G943

- **FINCHER, G.B. ; STONE, B.A. ; CLARKE, A.E.** Arabinogalactan-proteins: structure, biosynthesis, and function. *Ann. Rev. Plant Physiol.,* 1983, vol. 34, 47-70
- **FOOKS LJ ; FULLER R ; GIBSON GR.** Prebiotics, probiotics and human gut microbiology. *Int Dairy,* 1999, vol. J 9, 53-61
- **GDALA , J. ; JANSMAN .J.M. ; BURACZEWSKA, L. ; HUISMAN, J. ; VAN LEEUWEN, P.** The influence of alpha-galactosidase supplementation on the ileal digestibility of lupin seed carbohydrates and dietary protein in young pigs. *Anim. Feed Sci. Tech.,* 1997, vol. 67, 115-125
- **GEBOES K.P. ; DE PETER V ; LUYPAERTS A. ; BAMMENS B. ; EVENEPOEL P. ; GHOOS Y. ; RUTGEERTS P. ; VERBEKE K.** Validation of lactose[15N,15N]ureide as a tool to study colonic nitrogen metabolism. *Am J Physiol,* 2005, vol. 288, G994-G999
- **GIBSON , G.R. ; ROBERFROID M.B.** Dietary modulation of the human colonic microbiota: introducing the concept of prebiotics. *J. Nutr.,* 1995, vol. 125, 1401-1412
- **GRASTEN S ; LIUKKONEN K-H ; CHREVATIDIS A ; EL-NEZAMI H ; POUTANEN K ; MYKKANEN H.** Effects of wheat pentosan and inulin on the metabolic activity of fecal microbiota and on bowel function in healthy humans. *Nutrition Research,* 2003, vol. 23, 1503-1514
- **HOFMANN E.** Ueber den Abbau von glucoseureid durch Bakterien. *Biochem Zeitschr,* 1931, vol. 243, 416-422
- **HOSENY, R.C.** Principals of cereal science and technology. *AACC,* 1994
- **HSU C-K ; LIAO, J-W ; CHUNG, Y-C ; HSIEH, C-P ; CHAN, Y-C.** Xylooligosaccharides and fructooligosaccharides affect the intestinal microbiota and precancerous colonic lesions development in rats. *J. Nutr.,* 2004, vol. 134, 1523-1528
- **IJI, P.A.** The impact of cereal non-starch polysaccharides on intestinal development and function in broiler chickens. *Wld.'s Poult. Sci. J.,* 1999, vol. 55, 375-387
- **KABEL, MA. ; KORTENOEVEN, L. ; SCHOLS, H.A. ; VORAGEN, A.G.J.** In vitro fermentability of differently substituted xylo-oligosaccharides. *Journal of Agricultural and Food Chemistry,* 2002, vol. 50, 6205-6210
- **KOCHER, A. ; HUGHES, R.J. ; CHOCT, M.** Lupin oligosaccharides: nutrients and anti-nutrients?. *Proc. Of Australian Poultr. Sci. Symp.,* 1999
- **MAES, C. ; VANGENEUGDEN, B. ; DELCOUR, J.A.** Relative activity of two endoxylanases towards water-unextractable arabinoxylans in wheat bran. *J. Cereal Sci.,* 2004, vol. 39, 181-186
- **MCRITCHIE, F.** Studies of the methodology for fractionation and reconstitution of wheat flour. *J. Cereal Sci.,* 1985, vol. 3, 221-230
- **MORRIS, E.R.** Rheology of hydrocolloids in gums and stabilisers for the food industry. Pergamon Press, 1984
- **NAVEED, A.** The effect of enzyme supplementation of UK grown lupin seeds on growth and nutrient digestibility in broilers. *MSc. Thesis,* 1999
- **OKAZAKI M. ; FUJIKAWA, S. ; MATSUMOTO, N.** Effect of xylooligosaccharides on the growth of bifidobacteria. *Bifidobacteria Microflora,* 1990, vol. 9, 77-86
- **RADA, V. ; PETR, J.** A new selective medium for the isolation of glucose non-fermenting bifidobacteria from hen caeca. *Journal of Microbiological Methods,* 2000, vol. 43, 127-132
- **RADA, V. ; SIROTEK, K. ; PETR, J.** Evaluation of selective media for bifodobacteria in poultry and rabbit caecal samples. *Journal of Veterinary Medicine B,* 1999, vol. 46, 369-373
- **ROBERFROID, M.B.** Prebiotics and synbiotics: concepts and nutritional properties. *Brit. J. Nutr.,* 1998, vol. 80, S197-S202
- **SAINI, H.S.** *Legum seed oligosaccharides Prc Rec Adv Res Antinutritive Fact Legume Seed,* 1989, 329-341
- **SANTORI J. ; POTTHAST A. ; ECKER A. ; SIXTA H. ; ROSENAU T. ; KOSMA P.** Alkaline degradation kinetics and CE-separation of cello- and xylo-oligomers. *Carbohydrate Research,* 2003, vol. 338, 1209-1216
- **SATOKARI, R.M. ; VAUGHAN, E.E. ; AKKERMANS, A.D.L. ; SAARELA, M. ; DE VOS, W.M.** Bifidobacterial diversity in human feces detected by genus-specific PCR and denaturing gradient gel electrophoresis. *Appl. Env. Micorbiol.,* 2001, vol. 67, 504-513
- **SAVORY, C.J.** Enzyme supplementation, degradation and metabolism of three U-14C-labelled cell-wall substrates in the fowl. *Br. J. Nutr,* 1992, vol. 67, 91-102
- **SAVORY, C.J.** *Gastro-intestinal morphology and absorption of monosaccharides in fowls conditioned to different types and levels of dietary fibre,* 1992
- **SMITH EA ; MACFARLANE GT.** Enumeration of human colonic bacteria producing phenolic and indolic compounds: effects of pH, carbohydrate availability and retetion time on dissimilatory aromatic amino acid metabolism. *J Applied Bacteriol.,* 1996, vol. 81, 288-302
- **SNEDECOR, G.W. ; W.G. COCHRAN.** Statistical methods. Iowa State University Press, 1989
- **SPRING P. ; WENK C. ; DAWSON K.A. ; NEWMAN K.E.** The effects of dietary mannaoligosaccharides on cecal parameters and the concentrations of enteric bacteria in the ceca of salmonella-challenged broiler chicks. *Poult Sci,* 2001, vol. 79, 205-211

- *Reference Manual. Statistical Graphics Corporation,* 1992
- **STRAHM, A. ; AMADO, R. ; NEUKOM, H.** Hydroxyproline-galactoside as a protein-polysaccharide linkage in a water soluble arabinogalactan-peptide from wheat endosperm. *Phytochem.,* 1981, vol. 20, 1061-1063
- **SUNTORY.** *Xylo-oligosaccharide, brochure and product sheet,* 2001
- **TEITELBAUM, J.E. ; WALKER, W.A.** Nutritional impact of pre- and probiotics as protective gastrointestinal organisms. *Annual Review of Nutrition,* 2002, vol. 22, 107-38
- **TOPPING DL ; CLIFTON PM.** Short-chain fatty acids and human colonic function: roles of resistant starch and nonstarch polysaccharides. *Physiol Rev,* 2001, vol. 81, 1031-1064
- **TOYODA, Y. ; HATAKANA, Y. ; SUWA, Y.** Effect of xylooligosaccharides on calcium absorption. *Proc. of 47th Annual Meeting of Jpn Soc Nutr Food Sci,* 1993, 109
- **VAHOUNY, G.V.** *Fed.Proc.,* 1982, vol. 41, 2801
- **VAN DE WIELE, T. ; BOON, N. ; POSSEMIERS, S. ; JACOBS, H. ; VERSTRAETE, W.** Prebiotic effects of chicory inulin in the simulator of the human intestinal microbial ecosystem. *FEMS Microbiol. Ecol.,* 2004, vol. 51, 143-153
- **VAN LOO, J.A.E.** Prebiotics promote good health. The basis, the potential, and the emerging evidence. *J Clin Gastroenterol,* 2004, vol. 38, S70-S75
- **VINKX, C.J.A. ; VAN NIEUWENHOVE, C.G. ; DELCOUR, J.A.** Physico-chemical and functional properties of rye nonstarch polysaccharides. III. Oxidative gelation of a fraction containing water soluble pentosans and proteins. *Cereal Chem.,* 1991, vol. 68, 617-622
- **VISEK WJ.** Diet and cell growth modulation by ammonia. *Am J Clin Nutr,* 1978, vol. 31 (10), S216-S220
- **WHISTLER, R.L. ; BEMILLER, J.N.** Alkaline degradation of polysaccharides. *Adv. Carbohydr. Chem.,* 1958, vol. 13, 289-389
- **WUTZKE KD ; HEINE WE ; PLATH C ; LEITZMANN P ; RADKE M ; MOHR C ; RICHTER I ; GULZOW HU ; HOBUSCH D.** Evaluation of OCTT: a comparison of the lactose-[13C,15N]-ureide, CO2- and the lactulose H2-breath test in humans. *Eur J Clin Nutr,* 1997, vol. 51, 11-19
- **YAMADA H. ; ITOH, K. ; MORISHITA, Y. ; TANIGUCHI, H.** Structure and properties of oligosaccharides from wheat bran. *Cereal Foods World,* 1993, vol. 38, 490-492
- **ZDUNCZYK, Z. ; JUSKIEWICZ, J. ; FREJNAGEL S. ; GULEWICZ, K.** Influence of alkaloids and oligosaccha-rides from white lupin seeds utilisation of diets by rats and absorption of nutrients in the small intestine. *Anim. Feed Sci. Tech.,* 1998, vol. 72, 143-154
- **ZYLA, K. ; GOGAL, D. ; KORELESKI, J. ; SWIATKIEWICZ, S ; LEDOUX, D.R.** Simultaneous application of phytase and xylanase to broiler feeds based on wheat: feeding experiment with growing broilers. *J. Sci. Food Agric.,* 1999, vol. 79, 1841-1848
- **ZYLA , K. ; GOGAL, D. ; KORELESKI, J. ; SWIATKIEWICZ, S. ; LEDOUX, D.R.** Simultaneous application of phyatse and xylanase to broiler feeds based on wheat: in vitro measurements of phosphorus and pentose release from wheat and wheat-based feeds. *J. Sci. Food Agric.,* 1999, vol. 79, 1832-1840